(19) 
**Europäisches Patentamt**
**European Patent Office**
**Office européen des brevets**

(11) **EP 4 467 646 A1**

(12) **EUROPEAN PATENT APPLICATION**
published in accordance with Art. 153(4) EPC

(43) Date of publication:
**27.11.2024 Bulletin 2024/48**

(21) Application number: **23743341.2**

(22) Date of filing: **20.01.2023**

(51) International Patent Classification (IPC):
*C12N 15/12* (2006.01)　　*A61K 35/36* (2015.01)
*A61L 27/38* (2006.01)　　*A61L 27/50* (2006.01)
*A61L 27/60* (2006.01)　　*A61P 17/04* (2006.01)
*C12N 5/071* (2010.01)　　*C12N 5/10* (2006.01)
*C12N 15/63* (2006.01)　　*C12N 15/867* (2006.01)

(52) Cooperative Patent Classification (CPC):
**A61K 35/36; A61L 27/38; A61L 27/50; A61L 27/60;**
**A61P 17/04; C07K 14/435; C12N 5/06; C12N 5/10;**
**C12N 15/63; C12N 15/867**

(86) International application number:
**PCT/JP2023/001669**

(87) International publication number:
**WO 2023/140349 (27.07.2023 Gazette 2023/30)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **21.01.2022 JP 2022008057**

(71) Applicants:
- **OSAKA UNIVERSITY**
  **Suita-shi**
  **Osaka 565-0871 (JP)**
- **Stemrim Inc.**
  **Ibaraki-shi, Osaka 567-0085 (JP)**

(72) Inventors:
- **TAMAI, Katsuto**
  **Suita-shi, Osaka 565-0871 (JP)**
- **KIKUCHI, Yasushi**
  **Suita-shi, Osaka 565-0871 (JP)**
- **TAMAKOSHI, Tomoki**
  **Suita-shi, Osaka 565-0871 (JP)**
- **YAMAZAKI, Takehiko**
  **Ibaraki-shi, Osaka 567-0085 (JP)**

(74) Representative: **Doll, Markus Alexander**
**Kroher Strobel**
**Rechts-und Patentanwälte PartmbB**
**Bavariaring 20**
**80336 München (DE)**

Remarks:
The complete document including Reference
Table(s) and the Sequence Listing(s) can be
downloaded from the EPO website

(54) **CELL SHEET**

(57)　The present disclosure relates to a cell sheet for transplantation onto the skin of a patient with dystrophic epidermolysis bullosa. The cell sheet is composed of cells which are obtained from a body fluid within a blister of a patient with dystrophic epidermolysis bullosa. A type VII collagen gene is introduced into the cells.

EP 4 467 646 A1

## Description

TECHNICAL FIELD

The present application claims priority with respect to the Japanese Patent Application No. 2022-008057, which is incorporated herein by reference in its entirety.

The present disclosure relates to a cell sheet.

BACKGROUND ART

Epidermolysis bullosa is a disease in which adhesive structural molecules responsible for adhesion of the skin tissue are lost or disappeared, and then the epidermis peels off from the dermis and blisters or skin ulcers occur when force is applied to the skin. The disease includes simple epidermolysis bullosa, in which the epidermis is torn to form blisters, junctional epidermolysis bullosa, in which the epidermis is peeled from the basement membrane to form blisters, and dystrophic epidermolysis bullosa, in which the basement membrane is peeled from the dermis.

Dystrophic epidermolysis bullosa is the most common type of epidermolysis bullosa, accounting for about 50% of all epidermolysis bullosa. It is a hereditary disease caused by a mutation in the COL7A1 gene, which encodes type VII collagen. In the structure of the skin, the epidermal basal cells at the bottom of the epidermis are bound to a sheet-like structure called the basement membrane. Type VII collagen forms fibers called anchoring fibrils in the dermis and connects the basement membrane and the dermis. Therefore, if there is an abnormality in the type VII collagen gene, the adhesive function between the basement membrane and the dermis is impaired, resulting in dystrophic epidermolysis bullosa, in which blisters form between the basement membrane and the dermis. Among dystrophic epidermolysis bullosa, severe recessive dystrophic epidermolysis bullosa is a very serious hereditary bullous skin disease that has continued burn-like skin symptoms throughout the body immediately after birth, and cutaneous spinous cell carcinoma (scar cancer) occurs frequently from around 30 years old and leads to death.

There is currently no effective treatment for epidermolysis bullosa, and the development of gene therapy that radically suppresses blistering is required. As such gene therapy, a therapeutic technique is disclosed in which skin cells of a patient are collected, genetically engineered to produce type VII collagen, cultured to form a skin sheet, and transplanted into the patient (Patent Document 1). Also, it has been proposed to subject mesenchymal stem cells lacking the type VII collagen activity to genome editing, differentiate the mesenchymal stem cells capable of producing type VII collagen thus obtained into keratinocytes or fibroblasts, culture the cells to form a skin sheet, and use the skin sheet for treating a patient (see Patent Document 2).

PRIOR ART DOCUMENT

PATENT DOCUMENT

Patent Document 1: WO2017/120147
Patent Document 2: WO2018/154413

SUMMARY OF THE INVENTION

PROBLEMS TO BE SOLVED BY THE INVENTION

The present application provides a cell sheet which is effective for the treatment of dystrophic epidermolysis bullosa.

SOLUTIONS TO THE PROBLEMS

An aspect of the present invention is a cell sheet composed of cells which are obtained from a body fluid within a blister of a patient with dystrophic epidermolysis bullosa and into which a type VII collagen gene is introduced. This cell sheet is applied to the skin of a patient with dystrophic epidermolysis bullosa.

Another aspect is a method for producing a cell sheet, comprising first inoculating a body fluid within a blister of a patient with dystrophic epidermolysis bullosa into a medium; then obtaining cells adhering to the bottom of a culture vessel; then introducing a type VII collagen gene into the cells; then culturing the cells comprising the introduced type VII collagen gene; and finally taking out the cultured cells from a culture vessel as the cell sheet.

EFFECTS OF THE INVENTION

The present invention is useful for the treatment of dystrophic epidermolysis bullosa.

BRIEF DESCRIPTION OF THE DRAWINGS

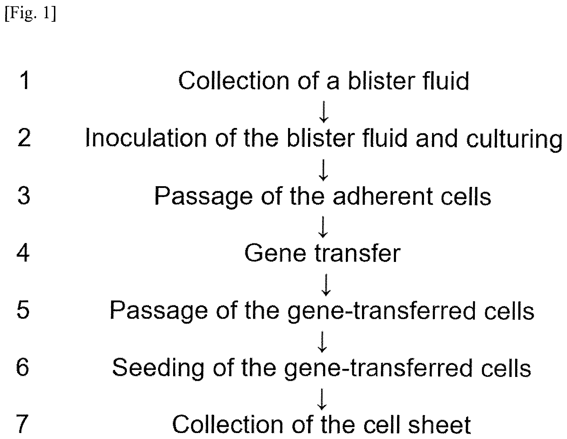

[Fig. 1] Fig. 1 shows a process diagram outlining the procedures to produce a cell sheet from blister-derived cells.

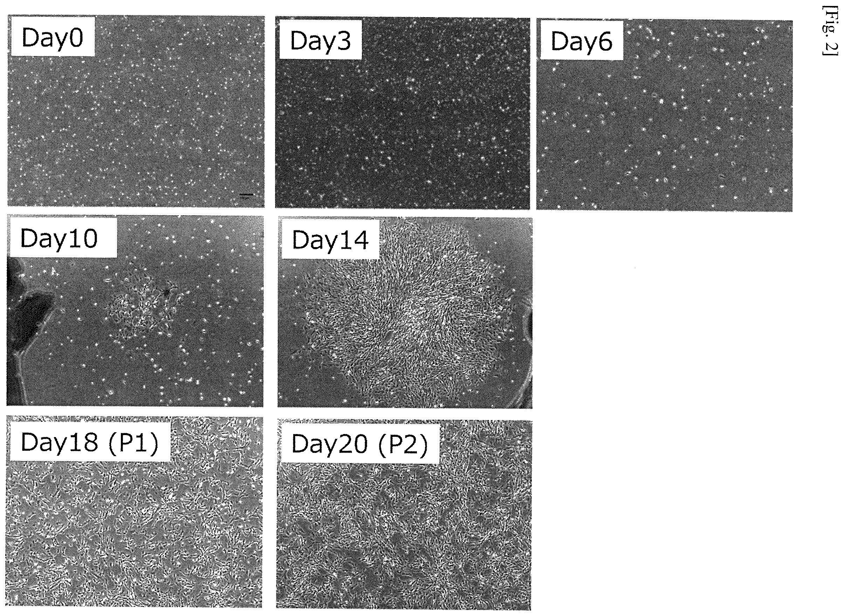

[Fig. 2] Fig. 2 is photographs showing the appearance of blister-derived cells up to 20 days after the initiation of culture.

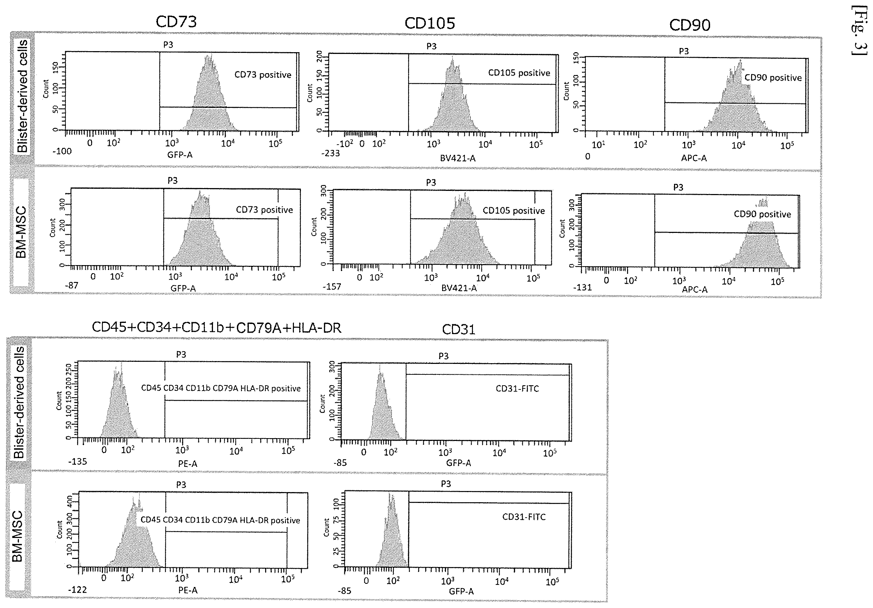

[Fig. 3] Fig. 3 shows the results of FACS analyses of blister-derived cells and human bone marrow-derived mesenchymal stem cells.

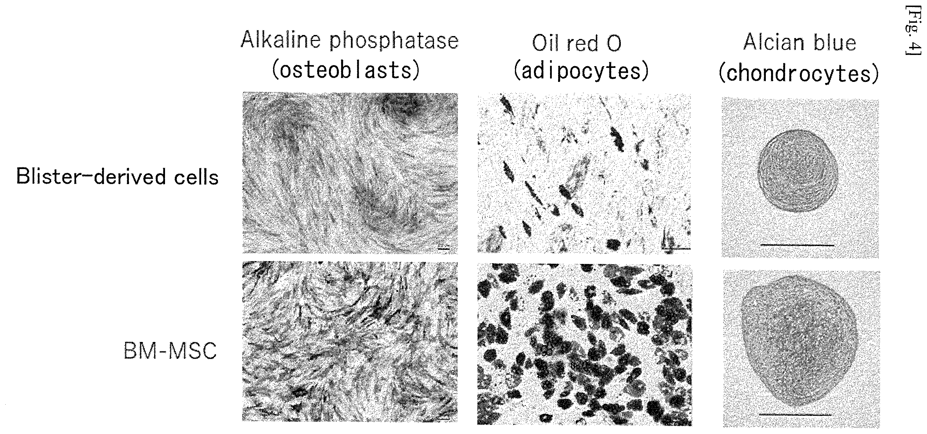

[Fig. 4] Fig. 4 shows blister-derived cells from a patient with dystrophic epidermolysis bullosa and human bone marrow-derived mesenchymal stem cells cultured under the condition that induces differentiation into osteoblasts, adipocytes, or chondrocytes. The results of Alkaline phosphatase (ALP) staining, Oil Red O staining, and Alcian Blue staining are shown.

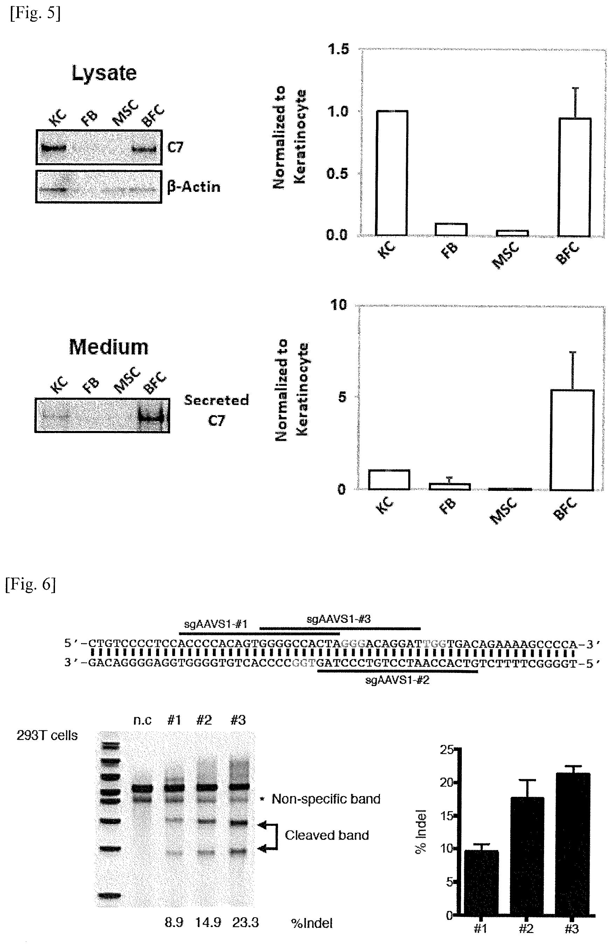

[Fig. 5] Fig. 5 shows the amounts of type VII collagen expressed or secreted by various cells. Fig. 5 (top) is a photograph showing the result of Western blotting of cell lysate using an anti-type VII collagen antibody (left), and a graph showing quantification of the densities of the obtained bands (right). Fig. 5 (bottom) is a photograph showing the result of Western blotting of the medium in which the cells were cultured (left), and a graph showing quantification of the densities of the obtained bands (right). The "KC" indicates human epidermal keratinocytes. The "FB" indicates human dermal fibroblasts. The "MSC" indicates human bone marrow-derived mesenchymal stem cells. The "BFC" indicates blister-derived cells.
[Fig. 6] Fig. 6 shows the cleavages of genomic DNA by designed sgRNAs (sgAAVS1-#1 to #3) and their cleavage efficiency.

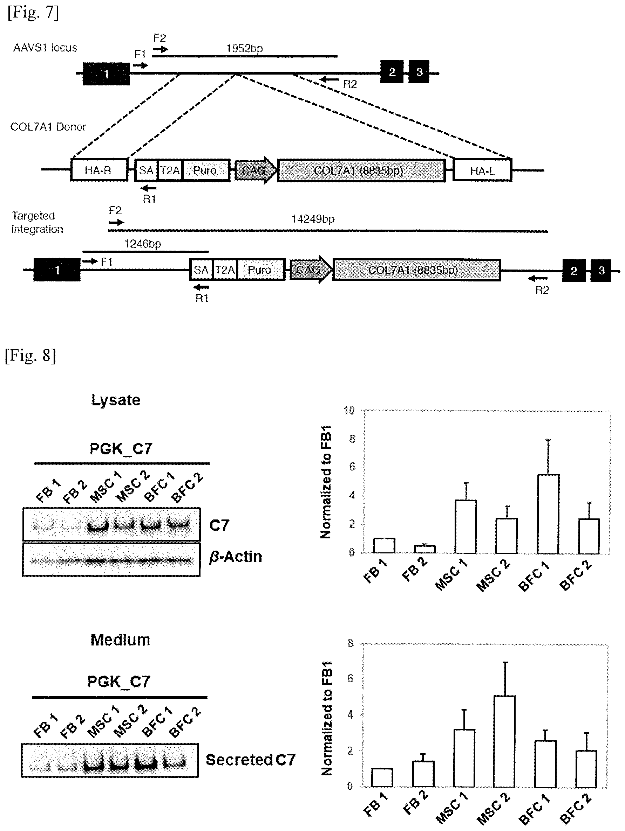

[Fig. 7] Fig. 7 is an explanatory diagram of genome editing in which a COL7A1 gene is introduced into the AAVS1 region. HA-R and HA-L indicate portions having homologous sequences, SA indicates a splice acceptor sequence, T2A indicates a T2A sequence encoding a T2A peptide, Puro indicates a puromycin resistance gene, and CAG indicates a CAG promoter sequence. The length from F2 to R2 in the wild-type genome (top) is 1952 bp, and the length from F1 to R1 and that from F2 to R2 in the genome into which the COL7A1 gene was introduced (bottom) is 1246 bp and 14249 bp, respectively.
[Fig. 8] Fig. 8 shows the amounts of type VII collagen expressed or secreted by various cells after the COL7A1 gene was introduced by CRISPR-Cas9. Fig. 8 (top) is a photograph showing the result of Western blotting of cell lysate using an anti-type VII collagen antibody (left), and a graph showing quantification of the densities of the obtained bands (right). Fig. 8 (bottom) is a photograph showing the result of Western blotting of the medium in which the cells were cultured (left), and a graph showing quantification of the densities of the obtained bands (right). The "FB" indicates human dermal fibroblasts. The "MSC" indicates human bone marrow-derived mesenchymal stem cells. The "BFC" indicates blister-derived cells.

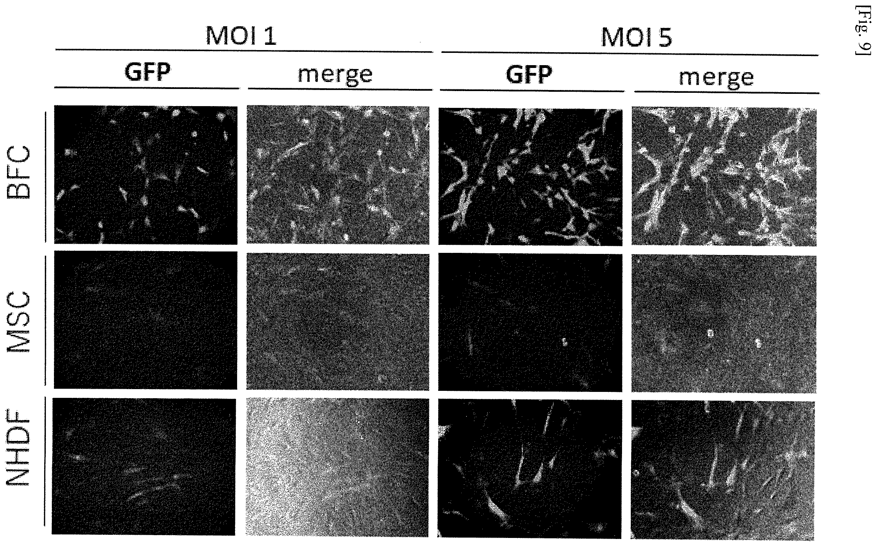

[Fig. 9] Fig. 9 shows fluorescence micrographs 72 hours after the infection of a GFP lentiviral vector to various cells. The "GFP" means a photograph showing green fluorescence of the cells. The "merge" means a superimposition of the bright field photograph on the "GFP" photograph. For "MOI 1", cells were infected with the lentiviral vector at MOI 1. For "MOI 5", cells were infected with the lentiviral vector at MOI 5. The "BFC" is a photograph of blister-derived cells, the "MSC" is a photograph of human bone marrow-derived mesenchymal stem cells, and the "NHDF" is a photograph of normal human adult dermal fibroblasts.

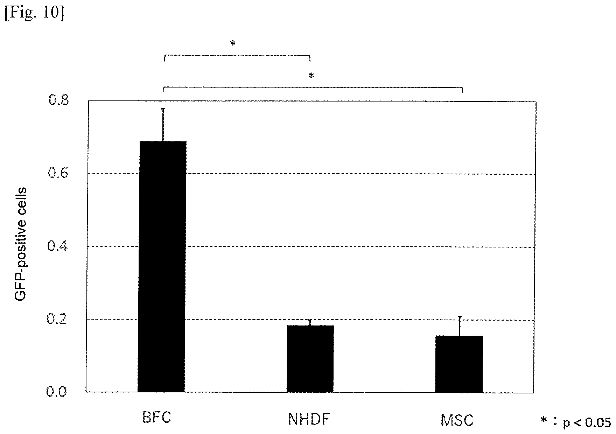

[Fig. 10] Fig. 10 is a graph showing the GFP positive cell ratios 72 hours after the infection with a GFP lentiviral vector at MOI 1 for each cell type.

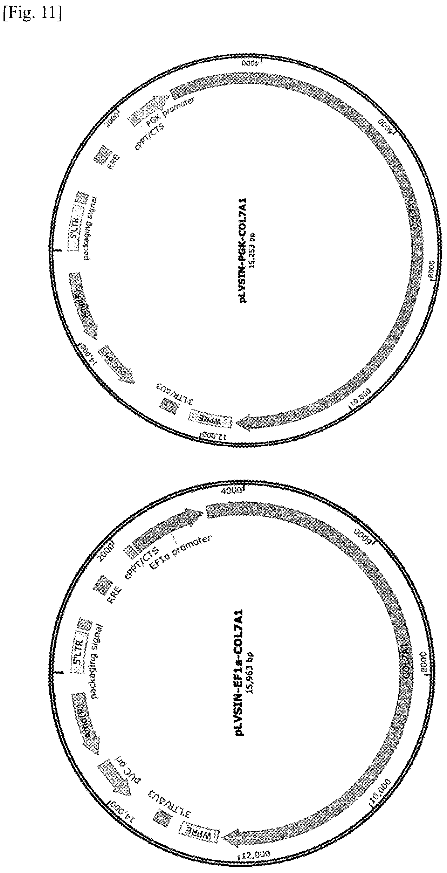

[Fig. 11] Fig. 11 is plasmid maps. Fig. 11 (left) shows the structure of the plasmid pLVSIN-EF1α-COL7A1 constructed by the present inventors. Fig. 11 (right) shows the structure of the plasmid pLUSIN-PGK-COL7A1 constructed by the present inventors. The pLVSIN vector is a SIN (self-inactivating) lentiviral vector plasmid, and the vector expresses a human type VII collagen gene under the control of an EF1α promoter (left) or a PGK promoter (right).

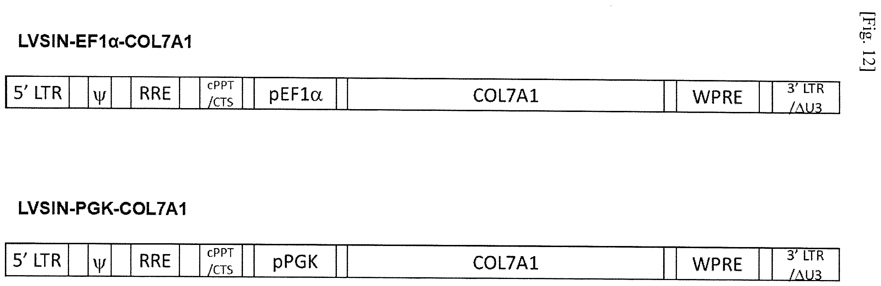

[Fig. 12] Fig. 12 is schematic diagrams showing the gene structures of the lentiviral vectors produced by the present inventors. The LVSIN-EF1α-COL7A1 vector shown in Fig. 12 (top) has an EF1α promoter and a COL7A1 gene in its expression cassette. The LVSIN-PGK-COL7A1 vector shown in Fig. 12 (bottom) has a PGK promoter and a COL7A1 gene in its expression cassette. In addition to HIV-1 LTR (5'LTR and 3'LTR/ΔU3) and the packaging signal (ψ), these vectors contain WPRE (woodchuck hepatitis virus post-transcriptional regulatory element), cPPT/CTS (central polypurine sequence/central termination sequence), and RRE (Rev response element) to improve transgene expression and viral titer.

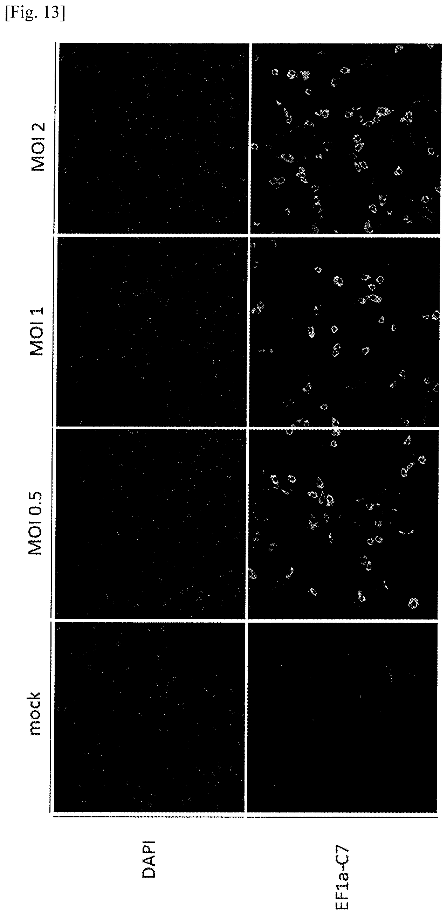

[Fig. 13] Fig. 13 is photographs of blister-derived cells immunostained with an anti-type VII collagen antibody 14 days after the infection with the EF1α-COL7A1 lentiviral vector. The "DAPI" is a photograph of DAPI staining. The "EF1a-C7" is a photograph showing the result of immunostaining for type VII collagen. For "mock", cells were not infected with any lentiviral vector. For "MOI 0.5", "MOI 1" and "MOI 2", cells were infected with the lentiviral vector at MOI 0.5, MOI 1 and MOI 2, respectively.

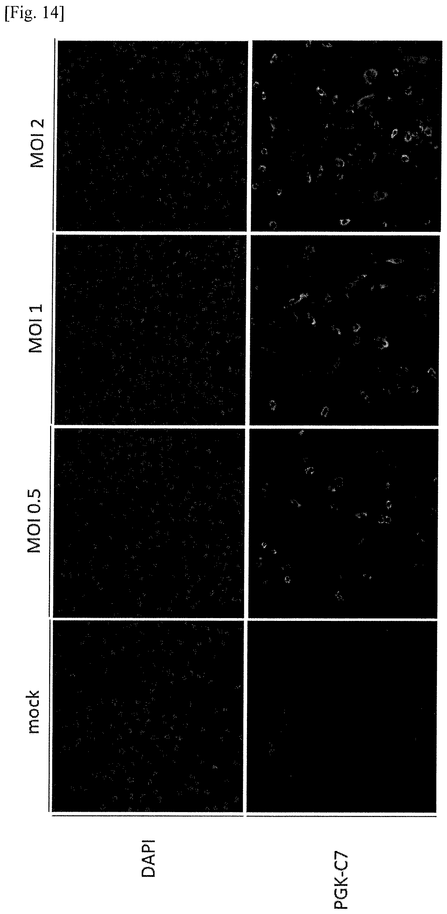

[Fig. 14] Fig. 14 is photographs of blister-derived cells immunostained with an anti-type VII collagen antibody 14 days after the infection with the PGK-COL7A1 lentiviral vector. The "DAPI" is a photograph of DAPI staining. The "PGK-C7" is a photograph showing the result of immunostaining for type VII collagen. For "mock", cells were not infected with any lentiviral vector. For "MOI 0.5", "MOI 1" and "MOI 2", cells were infected with the lentiviral vector at MOI 0.5, MOI 1 and MOI 2, respectively.

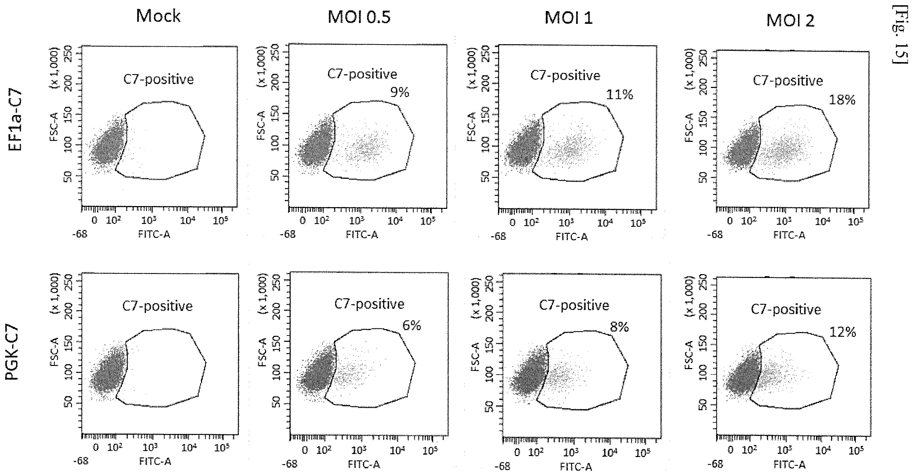

[Fig. 15] Fig. 15 shows the results of FACS analyses with an anti-type VII collagen antibody 14 days after the infection of blister-derived cells with a lentiviral vector having a type VII collagen gene. For "EF1a-C7", blister-derived cells were infected with the lentiviral vector containing an EF1α promoter and a COL7A1 gene in the expression cassette. For "PGK-C7", blister-derived cells were infected with the lentiviral vector containing a PGK promoter and a COL7A1 gene in the expression cassette. For "mock", cells were not infected with any lentiviral vector. For "MOI 0.5", "MOI 1", and "MOI 2", cells were infected with the lentiviral vector at MOI 0.5, MOI 1, and MOI 2, respectively.

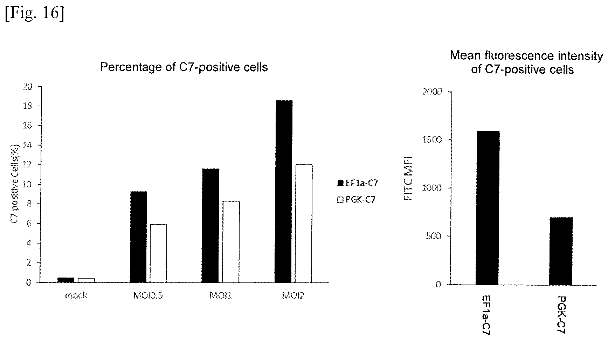

[Fig. 16] Fig. 16 is graphs showing quantification of the FACS data of Fig. 15. Fig. 16 (left) is a bar graph showing the percentage of type VII collagen-positive cells. Fig. 16 (right) is a bar graph showing the mean fluorescence intensity of type VII collagen-positive cells.

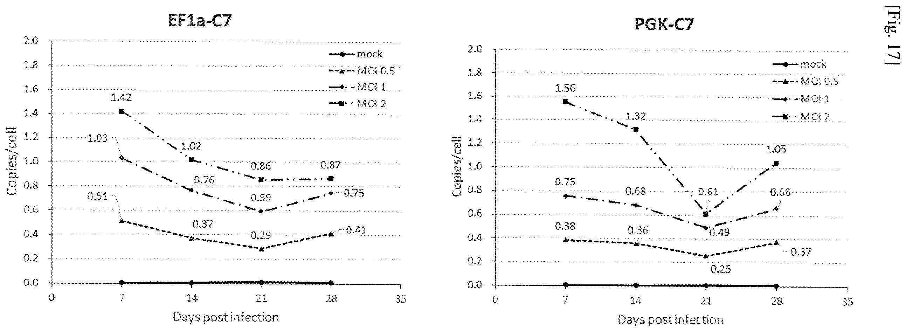

[Fig. 17] Fig. 17 is graphs showing the time-dependent change of vector copy number in the genome of blister-derived cells infected with a lentiviral vector having a type VII collagen gene.

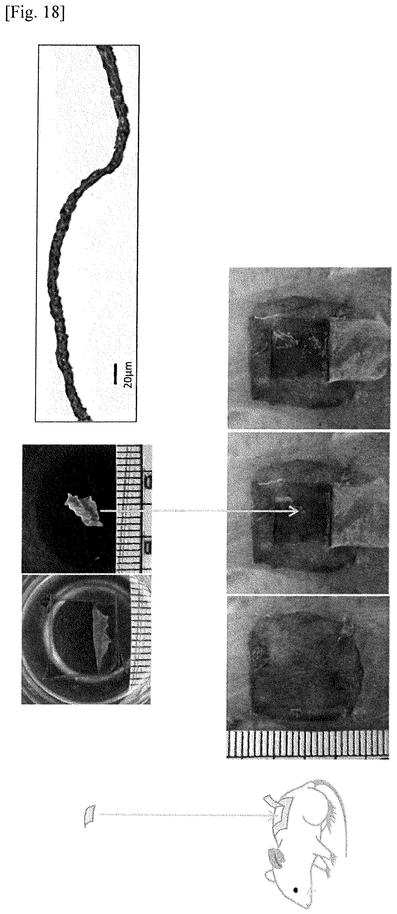

[Fig. 18] Fig. 18 (top) is photographs of the produced cell sheet. Fig. 18 (bottom) shows an illustration and photographs showing the procedures for transplanting the produced cell sheet onto an epidermolysis bullosa model mouse. Fig. 18 (top left) is a photograph of the formed cell sheet which is in the process of being detached from the plate. Fig. 18 (top middle) is a photograph of the cell sheet detached from the plate. Fig. 18 (top right) is a micrograph of the longitudinal section of the produced cell sheet stained with hematoxylin-eosin. Fig. 18 (bottom left) is an illustration of the procedure for transplanting the produced cell sheet onto an epidermolysis bullosa model mouse. Fig. 18 (second from the left at the bottom) is a photograph of a skin lesion of an epidermolysis bullosa model mouse. Fig. 18 (second from the right at the bottom) is a photograph in which the dermis was exposed after the epidermis of the epidermolysis bullosa model mouse was cut and opened. Fig. 18 (bottom right) is a photograph taken immediately after applying the produced cell sheet to the wound of the epidermolysis bullosa model mouse.

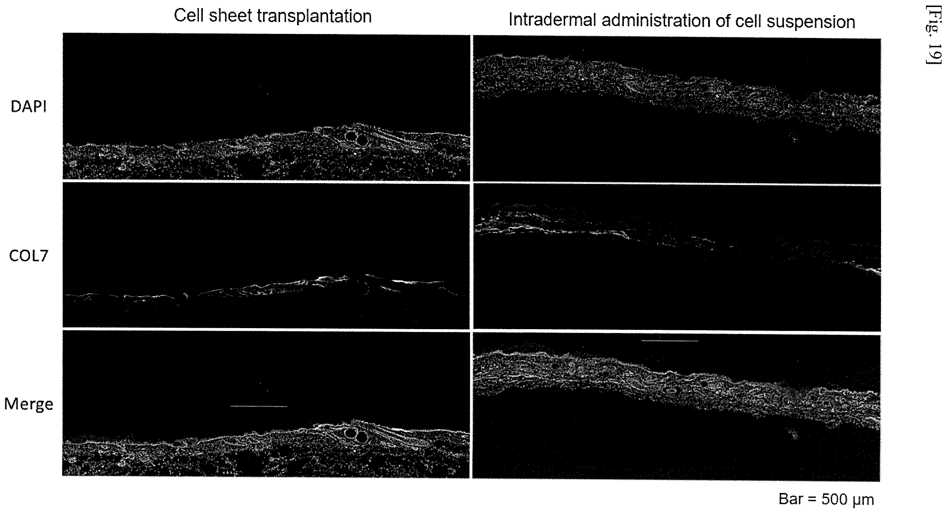

[Fig. 19] Fig. 19 shows sectional images of the skin of an epidermolysis bullosa model mouse with the cell sheet transplanted onto the skin lesion. The "DAPI" is a photograph of DAPI staining. The "COL7" is a photograph showing the result of immunostaining for type VII collagen. The "Merge" is a merged image of DAPI staining and immunostaining for type VII collagen. The "Cell sheet transplantation" shows the results of transplanting the cell sheet onto the skin lesion. This cell sheet was produced from the blister-derived cells into which a COL7A1 gene was introduced by CRISPR-Cas9. The "Intradermal administration of cell suspension" shows the results of intradermal administration of a suspension of the blister-derived cells into the skin lesion. A COL7A1 gene was introduced into the blister-derived cells by CRISPR-Cas9.

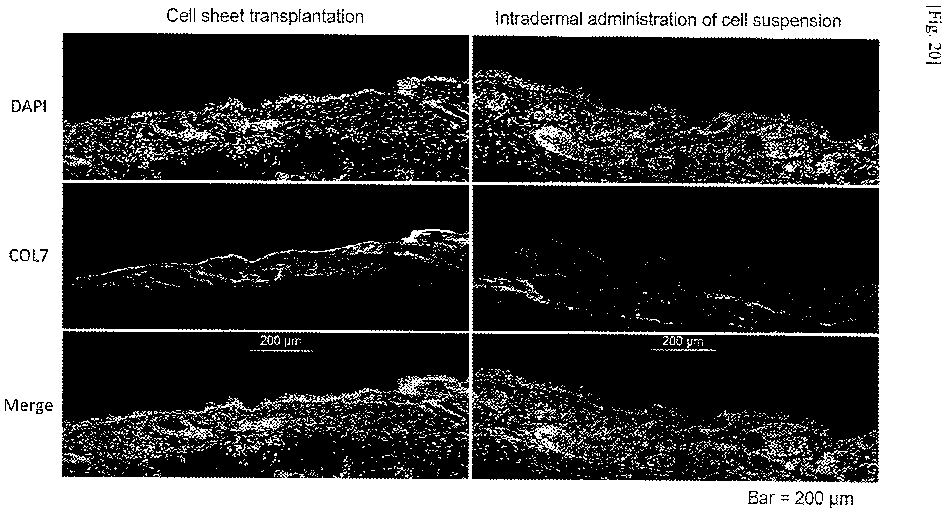

[Fig. 20] Fig. 20 shows micrographs showing the same results as those in Fig. 19 at a higher magnification than in Fig. 19.

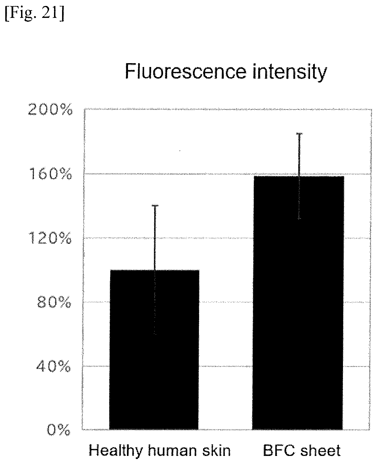

[Fig. 21] Fig. 21 is a graph showing the fluorescence intensity after type VII collagen immunostaining was performed on the skin of an epidermolysis bullosa model mouse 4 weeks after the transplantation of the blister-derived cell sheet onto the skin lesion.

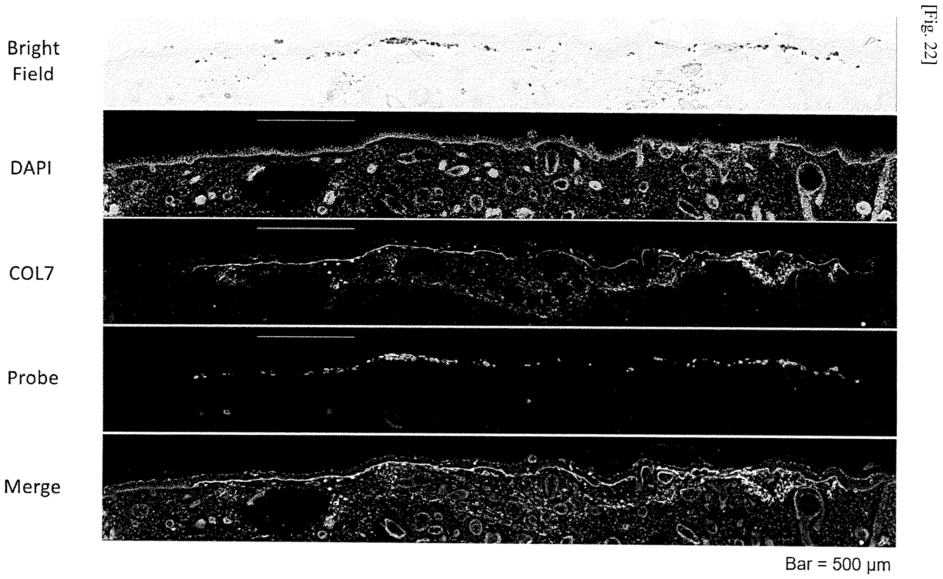

[Fig. 22] Fig. 22 shows photographs of in situ hybridization and immunostaining on the skin of an epidermolysis bullosa model mouse in which the cell sheet was transplanted onto the skin lesion. The "Bright Field" is a bright field photograph in which a probe complementary to type VII collagen mRNA is visualized. The "DAPI" is a photograph of DAPI staining. The "COL7" is a photograph showing the result of immunostaining for type VII collagen. The "Probe" shows the fluorescent labeling of the probe. The "Merge" is a merged image of DAPI staining and immunostaining for type VII collagen.

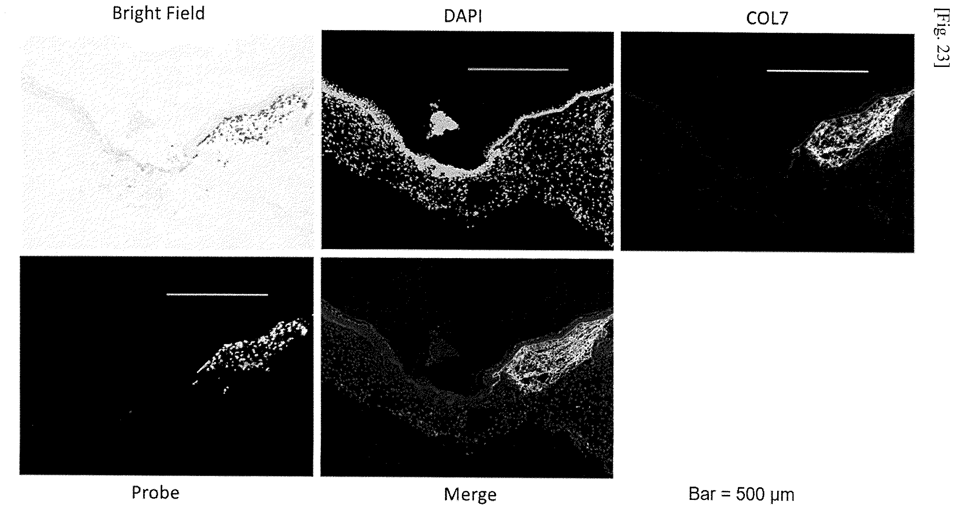

[Fig. 23] Fig. 23 shows the results of the same experiment as in Fig. 22 performed with a different epidermolysis bullosa model mouse.

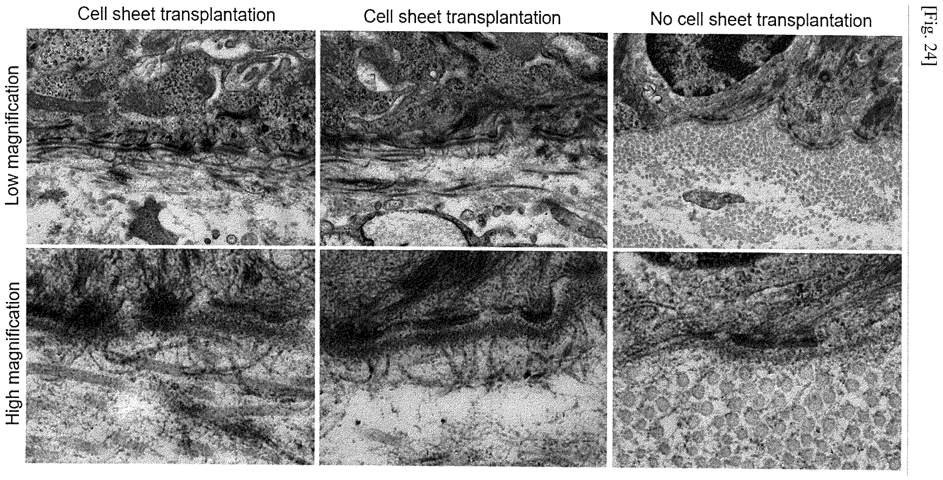

[Fig. 24] Fig. 24 shows electron micrographs of the skin of an epidermolysis bullosa model mouse in which the cell sheet was transplanted onto the skin lesion. The "Cell sheet transplantation" shows the results of transplanting the cell sheet onto the skin lesion. This cell sheet was produced from the blister-derived cells into which a COL7A1 gene was introduced by CRISPR-Cas9. The "No cell sheet transplantation" shows the skin lesion of the epidermolysis bullosa model mouse onto which cell sheet was not transplanted. The "Low magnification" is a photograph at 25300x. The short side of the "Low magnification" photo is 3.482 μm and the long side is 4.642 μm. The "High magnification" is a photograph at 84200x. The short side of the "High magnification" photo is 1.045 μm and the long side is 1.394 μm.

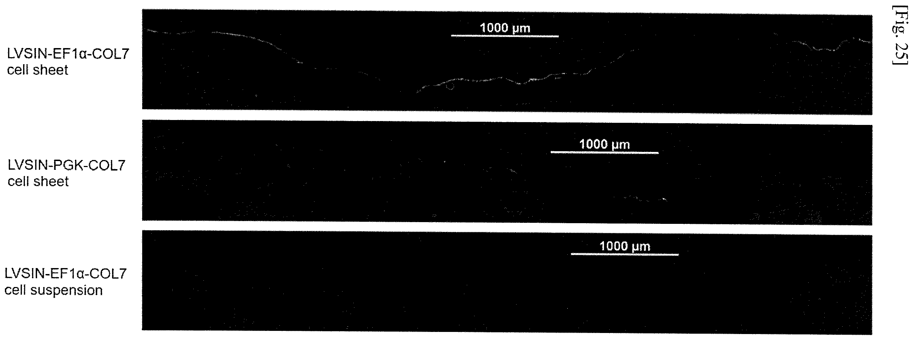

[Fig. 25] Fig. 25 shows sectional images of the skin of an epidermolysis bullosa model mouse in which a cell sheet was transplanted onto the skin lesion. The "LVSIN-EF1α-COL7 cell sheet" shows the result of transplanting the cell sheet onto the skin lesion. This cell sheet was produced from the blister-derived cells infected with the lentiviral vector carrying an EF1α promoter and a COL7A1 gene in its expression cassette. The "LVSIN-PGK-COL7 cell sheet" shows the result of transplanting the cell sheet onto the skin lesion. This cell sheet was produced from the blister-derived cells infected with the lentiviral vector carrying a PGK promoter and a COL7A1 gene in its expression cassette. The "LVSIN-EF1α-COL7 cell suspension" shows the result of intradermal administration of a suspension of the blister-derived cells into the skin lesion. These blister-derived cells were infected with the lentiviral vector carrying an EF1α promoter and a COL7A1 gene in its expression cassette.

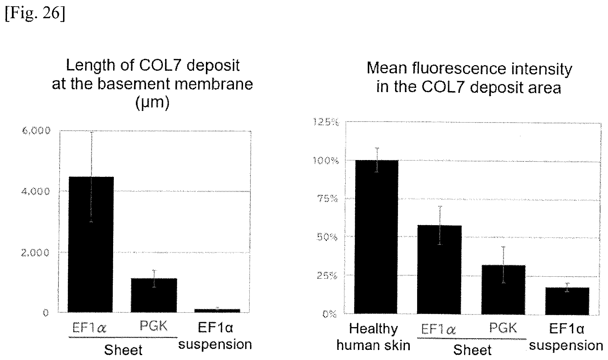

[Fig. 26] Fig. 26 (left) is a graph showing the length of type VII collagen deposit at the basement membrane 4 weeks after the transplantation of the cell sheet onto the skin lesion or 4 weeks after the intradermal administration of a suspension of blister-derived cells. The cell sheet was produced from the blister-derived cells into which a COL7A1 gene was introduced by a lentiviral vector. The COL7A1 gene was also introduced into the blister-derived cells for suspension by the lentiviral vector. Fig. 26 (right) is a graph showing the fluorescence intensity.

DETAILED DESCRIPTION

Unless otherwise specified, the terms used in the present disclosure have meanings generally understood by those skilled in the art in the fields such as organic chemistry, medical science, pharmaceutical science, molecular biology, and microbiology. Definitions of some terms used in the present disclosure are provided below, and these definitions supersede the general understandings in the present disclosure.

In one embodiment, the present disclosure provides a cell sheet. The cell sheet comprises cells which are derived from a body fluid within a blister of a patient with dystrophic epidermolysis bullosa. Into the cells, a type VII collagen gene is introduced. The cell sheet is transplanted onto the skin of a patient with dystrophic epidermolysis bullosa.

Preferably, a type VII collagen gene expression cassette is introduced into the cells. The type VII collagen gene expression cassette preferably comprises an EF1α promoter and a COL7A1 gene located downstream of the EF1α promoter.

In the cell sheet, the cells preferably overlap each other in the thickness direction, i.e., normal dirction, of the sheet. Also, in the cell sheet, the cells preferably form multiple layers. These features improve the mechanical strength of the cell sheet and provide the lesion with a larger number of cells per unit area. The cell sheet preferably has a thickness of about 5 to 50 μm, more preferably about 10 to 25 μm. This thickness ensures the mechanical strength of the cell sheet and allows sufficient oxygen to be provided to the cells inside the sheet.

Preferably, in a longitudinal section of the cell sheet, the cell sheet has a higher cell density near the surface than in the central portion of the sheet. More preferably, the cell sheet has a higher cell density on both the surfaces than in the central portion of the sheet. The cell sheet having a high cell density near the surface is strong and less easily break when it is peeled or transplaned. The central portion of the sheet may be a portion of the sheet that is located midway between one surface and the other.

Dystrophic epidermolysis bullosa (DEB) is a hereditary disease caused by a mutation in the COL7A1 gene, which encodes type VII collagen, and is known to be characterized in that no type VII collagen is produced or type VII collagen with reduced function due to the mutation is produced. The type VII collagen forms fibers called anchoring fibrils in the dermis and connects the basement membrane and the dermis. The type VII collagen contains a first non-collagen region, a collagen region, and a second non-collagen region from the N-terminus, and forms a triple chain at the collagen region, which is characterized by a repeating sequence of glycine-X-Y. Two molecules bind to each other at the C-terminus and the N-terminus binds to the basement membrane. Examples of the mutations include a mutation in which glycine in the collagen region is replaced by a different amino acid, a stop codon mutation that stops protein translation, and a splice site mutation. The mutation may be in one of the alleles or in both. Dystrophic epidermolysis bullosa includes dominant dystrophic epidermolysis bullosa and recessive dystrophic epidermolysis bullosa, and the recessive dystrophic epidermolysis bullosa includes severe generalized recessive dystrophic epidermolysis bullosa and other generalized types with relatively mild symptoms. The dystrophic epidermolysis bullosa herein may be any type of dystrophic epidermolysis bullosa, and the causal mutation in the COL7A1 gene may be any mutation.

A blister is an accumulation of fluid such as body fluid or tissue fluid beneath the epidermis. A preferable blister is a blister in which fluid is accumulated in the space formed between the epidermis and the dermis due to detachment of the epidermis from the dermis. More preferable blister is a blister in which fluid is accumulated in the space formed between the basement membrane of the epidermis and the dermis due to detachment of the basement membrane from the dermis.

In the present disclosure, a blister-derived cell of a patient with dystrophic epidermolysis bullosa refers to an adherent cell collected from a blister of a patient with dystrophic epidermolysis bullosa, and herein also referred to as "a DEB patient blister-derived cell" or "a blister-derived cell". The cell can be obtained by culturing a blister content of a patient with dystrophic epidermolysis bullosa on a solid surface. In one embodiment, the blister content is a fluid that has accumulated inside the blister (i.e., a body fluid within the blister), which is herein also referred to as "a blister fluid". The blister content can be collected from a blister of a patient with dystrophic epidermolysis bullosa with a tool such as a syringe. For example, a blister fluid can be aspirated into a syringe by piercing an injection needle of the syringe into a blister so that the tip of the injection needle is positioned in the space formed between the epidermis and the dermis and pulling the plunger of the syringe. In one embodiment, a blister-derived cell can be obtained by inoculating a blister content in a medium without any enzymatic treatment such as collagenase or dispase treatment and culturing it on a solid surface. Specifically, after a blister fluid collected from a blister is directly inoculated in a medium and the medium is incubated on a solid surface for a certain period of time, a cell adhering to the solid surface can be used as a blister-derived cell. In this case, it is preferable to obtain cells that have formed a colony on the solid surface. The blister fluid is preferably inoculated in the medium within 3 hours, more preferably within 2 hours, and even more preferably within 1 hour after the collection. In the present disclosure, a solid surface means a solid support to which a cell can adhere, and includes, for example, plastic or glass culture vessels such as culture dishes, flasks and multiwell plates. In one embodiment, the solid surface is a plastic culture vessel. The solid surface may be coated, and examples of coating substances include collagen I, laminin, vitronectin, fibronectin, poly-L-lysine, and poly-L-ornithine. In one embodiment, the solid surface is coated with collagen I. The culturing can be performed in a general incubator under the condition such as "37°C, 5% $CO_2$" or "37°C, 5% $O_2$, 5% $CO_2$". The culture medium may be any medium that can be used for culturing animal cells, and can be, for example, MEM, MEMα, DMEM, GMEM, RPMI 1640, MesenCult™ (STEMCELL Technologies), Mesenchymal Stem Cell Growth Medium 2 (PromoCell), MSCGM Mesenchymal Stem Cell Growth Medium (Lonza), Cellartis MSC Xeno-Free Culture Medium (Takara Bio), or a mixture thereof. Among them, a medium for culturing mesenchymal stem cells such as MesenCult™, Mesenchymal Stem Cell Growth Medium 2, MSCGM Mesenchymal Stem Cell Growth Medium, or Cellartis MSC Xeno-Free Culture Medium is preferably used. The medium is preferably a serum-free medium. The culture period may be any period sufficient for the cell to adhere to the solid surface, and can be e.g., 1 day to several months (for example, for 2, 3 or 4 months), 1 day to 1 month, 1 day to several weeks (for example, 2, 3 or 4 weeks), or 1 days to 1 week.

In an embodiment, the DEB patient blister-derived cell has one or more characteristics selected from:

1) adherent to a solid surface;
2) positive for one or more surface markers selected from the group consisting of CD73, CD105 and CD90;
3) negative for one or more surface markers selected from the group consisting of CD45, CD34, CD11b, CD79A, HLA-DR and CD31;
4) incapable of differentiating into an osteoblast, or less capable of differentiating into an osteoblast than a bone marrow-derived mesenchymal stem cell;
5) incapable of differentiating into an adipocyte, or less capable of differentiating into an adipocyte than a bone marrow-derived mesenchymal stem cell; and
6) incapable of differentiating into a chondrocyte, or less capable of differentiating into a chondrocyte than a bone marrow-derived mesenchymal stem cell.

In the present disclosure, the expression that a cell is "incapable of differentiating" into an osteoblast, an adipocyte or a chondrocyte means that differentiation of the cell into an osteoblast, an adipocyte or a chondrocyte cannot be detected by a standard detection method (such as staining) after induction of differentiation with a standard condition.

In an embodiment, the DEB patient blister-derived cell is a CD73-positive, CD105-positive, and CD90-positive cell. In another embodiment, the DEB patient blister-derived cell is a CD45-negative, CD34-negative, CD11b-negative, CD79A-negative, HLA-DR-negative, and CD31-negative cell. In a further embodiment, the DEB patient blister-derived cell is a cell that is less capable of differentiating into an osteoblast, an adipocyte and a chondrocyte than a bone marrow-derived mesenchymal stem cell.

In the present disclosure, a blister-derived cell of a patient with dystrophic epidermolysis bullosa that is genetically modified to produce type VII collagen is used. As used herein, the term "cell that is genetically modified to produce type VII collagen" means a cell that is genetically modified to produce a functional type VII collagen (i.e., a type VII collagen capable of forming anchoring fibrils).

In the present disclosure, genetic modification of a cell means both modification of a gene in the genome of the cell and modification of the cell to express a gene from a nucleic acid construct outside the genome (such as a vector). That is, the expression "genetically modifying a cell to produce type VII collagen" includes modifying a cell to express type VII collagen from a COL7A1 gene in the genome, and modifying a cell to express type VII collagen from a COL7A1 gene in a nucleic acid construct outside the genome. Also, "a cell that is genetically modified to produce type VII collagen" includes a cell that expresses type VII collagen from a COL7A1 gene in the genome and a cell that expresses type VII collagen from a COL7A1 gene in a nucleic acid construct outside the genome.

Genetic modification of a cell can be carried out by introducing a COL7A1 gene or by correcting a mutation in the COL7A1 gene in the genome. In one embodiment, the present disclosure uses cells into which a type VII collagen gene is introduced. The introduction of a COL7A1 gene can be carried out either by introducing a COL7A1 gene into the genome of the cell or by placing a nucleic acid construct comprising a COL7A1 gene in the cell so that the COL7A1 gene is expressed from the nucleic acid construct outside the genome. When a COL7A1 gene is introduced into the genome of a cell, the COL7A1 gene may be introduced at a specific site or may be introduced at random. In an embodiment, the COL7A1 gene is introduced into the COL7A1 locus of the genome, or a safe harbor such as the AAVS 1 region.

The DEB patient blister-derived cell may be a cell of a patient with dystrophic epidermolysis bullosa to which the cell is to be administered (ie, an autologous cell), or a cell obtained from a subject other than the patient (ie, an allogeneic cell). The cell of a patient with dystrophic epidermolysis bullosa includes a cell that does not produce type VII collagen and a cell that produces type VII collagen with reduced function due to a mutation, and the "cell of a patient with dystrophic epidermolysis bullosa" as used herein may be any of them.

The DEB patient blister-derived cell may be any cell as long as it produces type VII collagen in the vicinity of the epidermal basement membrane when administered to a patient.

In the present disclosure, the term "cell" is used in the sense of including a cell after proliferation as needed. Proliferation of a cell can be carried out by culturing the cell. For example, "a blister-derived cell (of a patient with dystrophic epidermolysis bullosa)" includes a cell that is proliferated from a cell collected from a patient, and "a genetically modified cell" includes a cell that is proliferated from a cell obtained by genetic modification. When genetic modification is carried out, a cell may be prolifelated until the amount required for the genetic modification is obtained. Also, after genetic modification, the cell may be prolifelated until the amount required for treatment is obtained.

As used herein, the term "cell" can mean a single cell or multiple cells, depending on the context. Further, the cell may be a cell population composed of one type of cell or a cell population including a plurality of types of cells.

As used herein, the term "type VII collagen gene" or "COL7A1 gene" means a nucleic acid sequence encoding type VII collagen, and is used to include cDNA as well as a sequence containing one or more introns (for example, a genomic sequence or a minigene). The representative nucleic acid sequence of the human COL7A1 gene (cDNA) is shown in SEQ ID NO: 1, and the representative amino acid sequence of human type VII collagen is shown in SEQ ID NO: 2. The cDNA sequence of the COL7A1 gene is disclosed in GenBank: NM_000094.3, and the genome sequence is disclosed in GenBank: AC121252.4. The sequence of a COL7A1 gene is not limited to any specific sequence as long as it encodes a functional type VII collagen (ie, a type VII collagen capable of forming anchoring fibrils).

cDNA sequence of human COL7A1 gene (8835bp) (SEQ ID NO: 1)

ATGACGCTGCGGCTTCTGGTGGCCGCGCTCTGCGCCGGGATCCTGGCAGAGG

CGCCCCGAGTGCGAGCCCAGCACAGGGAGAGAGTGACCTGCACGCGCCTTT

ACGCCGCTGACATTGTGTTCTTACTGGATGGCTCCTCATCCATTGGCCGCAGC

AATTTCCGCGAGGTCCGCAGCTTTCTCGAAGGGCTGGTGCTGCCTTTCTCTG

GAGCAGCCAGTGCACAGGGTGTGCGCTTTGCCACAGTGCAGTACAGCGATG

ATCCACGGACAGAGTTCGGCCTGGATGCACTTGGCTCTGGGGGTGATGTGAT

CCGCGCCATCCGTGAGCTTAGCTACAAGGGGGGCAACACTCGCACAGGGGC

TGCAATTCTCCATGTGGCTGACCATGTCTTCCTGCCCAGCTGGCCCGACCTG

GTGTCCCCAAGGTCTGCATCCTGATCACAGACGGGAAGTCCCAGGACCTGGT

GGACACAGCTGCCCAAAGGCTGAAGGGGCAGGGGGTCAAGCTATTTGCTGT

GGGGATCAAGAATGCTGACCCTGAGGAGCTGAAGCGAGTTGCCTCACAGCC

CACCAGTGACTTCTTCTTCTTCGTCAATGACTTCAGCATCTTGAGGACACTAC

TGCCCCTCGTTTCCCGGAGAGTGTGCACGACTGCTGGTGGCGTGCCTGTGAC

CCGACCTCCGGATGACTCGACCTCTGCTCCACGAGACCTGGTGCTGTCTGAG

CCAAGCAGCCAATCCTTGAGAGTACAGTGGACAGCGGCCAGTGGCCCTGTG

ACTGGCTACAAGGTCCAGTACACTCCTCTGACGGGGCTGGGACAGCCACTG

CCGAGTGAGCGGCAGGAGGTGAACGTCCCAGCTGGTGAGACCAGTGTGCGG

CTGCGGGGTCTCCGGCCACTGACCGAGTACCAAGTGACTGTGATTGCCCTCT

ACGCCAACAGCATCGGGGAGGCTGTGAGCGGGACAGCTCGGACCACTGCCC

TAGAAGGGCCGGAACTGACCATCCAGAATACCACAGCCCACAGCCTCCTGGT

GGCCTGGCGGAGTGTGCCAGGTGCCACTGGCTACCGTGTGACATGGCGGGT

CCTCAGTGGTGGGCCCACACAGCAGCAGGAGCTGGGCCCTGGGCAGGGTTC

AGTGTTGCTGCGTGACTTGGAGCCTGGCACGGACTATGAGGTGACCGTGAGC

ACCCTATTTGGCCGCAGTGTGGGGCCCGCCACTTCCCTGATGGCTCGCACTG

ACGCTTCTGTTGAGCAGACCCTGCGCCCGGTCATCCTGGGCCCCACATCCAT

CCTCCTTTCCTGGAACTTGGTGCCTGAGGCCCGTGGCTACCGGTTGGAATGG

CGGCGTGAGACTGGCTTGGAGCCACCGCAGAAGGTGGTACTGCCCTCTGAT

GTGACCCGCTACCAGTTGGATGGGCTGCAGCCGGGCACTGAGTACCGCCTCA

CACTCTACACTCTGCTGGAGGGCCACGAGGTGGCCACCCCTGCAACCGTGGT

TCCCACTGGACCAGAGCTGCCTGTGAGCCCTGTAACAGACCTGCAAGCCAC

CGAGCTGCCCGGGCAGCGGGTGCGAGTGTCCTGGAGCCCAGTCCCTGGTGC

CACCCAGTACCGCATCATTGTGCGCAGCACCCAGGGGGTTGAGCGGACCCTG

GTGCTTCCTGGGAGTCAGACAGCATTCGACTTGGATGACGTTCAGGCTGGGC

TTAGCTACACTGTGCGGGTGTCTGCTCGAGTGGGTCCCCGTGAGGGCAGTGC

CAGTGTCCTCACTGTCCGCCGGGAGCCGGAAACTCCACTTGCTGTTCCAGGG

CTGCGGGTTGTGGTGTCAGATGCAACGCGAGTGAGGGTGGCCTGGGGACCC

GTCCCTGGAGCCAGTGGATTTCGGATTAGCTGGAGCACAGGCAGTGGTCCGG

AGTCCAGCCAGACACTGCCCCCAGACTCTACTGCCACAGACATCACAGGGCT

GCAGCCTGGAACCACCTACCAGGTGGCTGTGTCGGTACTGCGAGGCAGAGA

GGAGGGCCCTGCTGCAGTCATCGTGGCTCGAACGGACCCACTGGGCCCAGT

GAGGACGGTCCATGTGACTCAGGCCAGCAGCTCATCTGTCACCATTACCTGG

ACCAGGGTTCCTGGCGCCACAGGATACAGGGTTTCCTGGCACTCAGCCCACG

GCCCAGAGAAATCCCAGTTGGTTTCTGGGGAGGCCACGGTGGCTGAGCTGG

ATGGACTGGAGCCAGATACTGAGTATACGGTGCATGTGAGGGCCCATGTGGC

TGGCGTGGATGGGCCCCCTGCCTCTGTGGTTGTGAGGACTGCCCCTGAGCCT

GTGGGTCGTGTGTCGAGGCTGCAGATCCTCAATGCTTCCAGCGACGTTCTAC

GGATCACCTGGGTAGGGGTCACTGGAGCCACAGCTTACAGACTGGCCTGGG

GCCGGAGTGAAGGCGGCCCCATGAGGCACCAGATACTCCCAGGAAACACAG

ACTCTGCAGAGATCCGGGGTCTCGAAGGTGGAGTCAGCTACTCAGTGCGAG

TGACTGCACTTGTCGGGGACCGCGAGGGCACACCTGTCTCCATTGTTGTCAC

TACGCCGCCTGAGGCTCCGCCAGCCCTGGGGACGCTTCACGTGGTGCAGCG

CGGGGAGCACTCGCTGAGGCTGCGCTGGGAGCCGGTGCCCAGAGCGCAGG

GCTTCCTTCTGCACTGGCAACCTGAGGGTGGCCAGGAACAGTCCCGGGTCCT

GGGGCCCGAGCTCAGCAGCTATCACCTGGACGGGCTGGAGCCAGCGACACA

GTACCGCGTGAGGCTGAGTGTCCTAGGGCCAGCTGGAGAAGGGCCCTCTGC

AGAGGTGACTGCGCGCACTGAGTCACCTCGTGTTCCAAGCATTGAACTACGT

GTGGTGGACACCTCGATCGACTCGGTGACTTTGGCCTGGACTCCAGTGTCCA

GGGCATCCAGCTACATCCTATCCTGGCGGCCACTCAGAGGCCCTGGCCAGGA

AGTGCCTGGGTCCCCGCAGACACTTCCAGGGATCTCAAGCTCCCAGCGGGT

GACAGGGCTAGAGCCTGGCGTCTCTTACATCTTCTCCCTGACGCCTGTCCTG

GATGGTGTGCGGGGTCCTGAGGCATCTGTCACACAGACGCCAGTGTGCCCCC

GTGGCCTGGCGGATGTGGTGTTCCTACCACATGCCACTCAAGACAATGCTCA

CCGTGCGGAGGCTACGAGGAGGGTCCTGGAGCGTCTGGTGTTGGCACTTGG

GCCTCTTGGGCCACAGGCAGTTCAGGTTGGCCTGCTGTCTTACAGTCATCGG

CCCTCCCCACTGTTCCCACTGAATGGCTCCCATGACCTTGGCATTATCTTGCA

AAGGATCCGTGACATGCCCTACATGGACCCAAGTGGGAACAACCTGGGCAC

AGCCGTGGTCACAGCTCACAGATACATGTTGGCACCAGATGCTCCTGGGCGC

CGCCAGCACGTACCAGGGGTGATGGTTCTGCTAGTGGATGAACCCTTGAGAG

GTGACATATTCAGCCCCATCCGTGAGGCCCAGGCTTCTGGGCTTAATGTGGTG

ATGTTGGGAATGGCTGGAGCGGACCCAGAGCAGCTGCGTCGCTTGGCGCCG

GGTATGGACTCTGTCCAGACCTTCTTCGCCGTGGATGATGGGCCAAGCCTGG

ACCAGGCAGTCAGTGGTCTGGCCACAGCCCTGTGTCAGGCATCCTTCACTAC

TCAGCCCCGGCCAGAGCCCTGCCCAGTGTATTGTCCAAAGGGCCAGAAGGG

GGAACCTGGAGAGATGGGCCTGAGAGGACAAGTTGGGCCTCCTGGCGACCC

TGGCCTCCCGGGCAGGACCGGTGCTCCCGGCCCCCAGGGGCCCCTGGAAG

TGCCACTGCCAAGGGCGAGAGGGGCTTCCCTGGAGCAGATGGGCGTCCAGG

CAGCCCTGGCCGCGCCGGGAATCCTGGGACCCCTGGAGCCCCTGGCCTAAA

GGGCTCTCCAGGGTTGCCTGGCCCTCGTGGGGACCCGGGAGAGCGAGGACC

TCGAGGCCCAAAGGGGGAGCCGGGGGCTCCCGGACAAGTCATCGGAGGTG

AAGGACCTGGGCTTCCTGGGCGGAAAGGGGACCCTGGACCATCGGGCCCCC

CTGGACCTCGTGGACCACTGGGGGACCCAGGACCCCGTGGCCCCCCAGGGC

TTCCTGGAACAGCCATGAAGGGTGACAAAGGCGATCGTGGGGAGCGGGGTC

CCCCTGGACCAGGTGAAGGTGGCATTGCTCCTGGGGAGCCTGGGCTGCCGG

GTCTTCCCGGAAGCCCTGGACCCCAAGGCCCCGTTGGCCCCCCTGGAAAGA

AAGGAGAAAAGGTGACTCTGAGGATGGAGCTCCAGGCCTCCCAGGACAAC

CTGGGTCTCCGGGTGAGCAGGGCCCACGGGGACCTCCTGGAGCTATTGGCCC

CAAAGGTGACCGGGGCTTTCCAGGGCCCTGGGTGAGGCTGGAGAGAAGG

GCGAACGTGGACCCCCAGGCCCAGCGGGATCCCGGGGGCTGCCAGGGGTTG

CTGGACGTCCTGGAGCCAAGGGTCCTGAAGGGCCACCAGGACCCACTGGCC

GCCAAGGAGAGAAGGGGGAGCCTGGTCGCCCTGGGGACCCTGCAGTGGTG

GGACCTGCTGTTGCTGGACCCAAAGGAGAAAAGGGAGATGTGGGGCCCGCT

GGGCCCAGAGGAGCTACCGGAGTCCAAGGGGAACGGGGCCCACCCGGCTTG

GTTCTTCCTGGAGACCCTGGCCCCAAGGGAGACCCTGGAGACCGGGGTCCC

ATTGGCCTTACTGGCAGAGCAGGACCCCCAGGTGACTCAGGGCCTCCTGGA

GAGAAGGGAGACCCTGGGCGGCCTGGCCCCCCAGGACCTGTTGGCCCCCGA

GGACGAGATGGTGAAGTTGGAGAGAAAGGTGACGAGGGTCCTCCGGGTGA

CCCGGGTTTGCCTGGAAAAGCAGGCGAGCGTGGCCTTCGGGGGGCACCTGG

AGTTCGGGGGCCTGTGGGTGAAAAGGGAGACCAGGGAGATCCTGGAGAGG

ATGGACGAAATGGCAGCCCTGGATCATCTGGACCCAAGGGTGACCGTGGGG

AGCCGGGTCCCCCAGGACCCCGGGACGGCTGGTAGACACAGGACCTGGAG

CCAGAGAGAAGGGAGAGCCTGGGGACCGCGGACAAGAGGGTCCTCGAGGG

CCCAAGGGTGATCCTGGCCTCCCTGGAGCCCCTGGGGAAAGGGGCATTGAA

GGGTTTCGGGGACCCCCAGGCCCACAGGGGGACCCAGGTGTCCGAGGCCCA

GCAGGAGAAAAGGGTGACCGGGGTCCCCCTGGGCTGGATGGCCGGAGCGG

ACTGGATGGGAAACCAGGAGCCGCTGGGCCCTCTGGGCCGAATGGTGCTGC

AGGCAAAGCTGGGGACCCAGGGAGAGACGGGCTTCCAGGCCTCCGTGGAG

AACAGGGCCTCCCTGGCCCCTCTGGTCCCCCTGGATTACCGGGAAAGCCAGG

CGAGGATGGCAAACCTGGCCTGAATGGAAAAAACGGAGAACCTGGGGACCC

TGGAGAAGACGGGAGGAAGGGAGAGAAGGAGATTCAGGCGCCTCTGGGA

GAGAAGGTCGTGATGGCCCCAAGGGTGAGCGTGGAGCTCCTGGTATCCTTGG

ACCCCAGGGGCCTCCAGGCCTCCCAGGGCCAGTGGGCCCTCCTGGCCAGGG

TTTTCCTGGTGTCCCAGGAGGCACGGGCCCCAAGGGTGACCGTGGGGAGAC

TGGATCCAAAGGGGAGCAGGGCCTCCCTGGAGAGCGTGGCCTGCGAGGAGA
GCCTGGAAGTGTGCCGAATGTGGATCGGTTGCTGGAAACTGCTGGCATCAAG
GCATCTGCCCTGCGGGAGATCGTGGAGACCTGGGATGAGAGCTCTGGTAGCT
TCCTGCCTGTGCCCGAACGGCGTCGAGGCCCCAAGGGGGACTCAGGCGAAC
AGGGCCCCCCAGGCAAGGAGGGCCCCATCGGCTTTCCTGGAGAACGCGGGC
TGAAGGGCGACCGTGGAGACCCTGGCCCTCAGGGGCCACCTGGTCTGGCCC
TTGGGGAGAGGGGCCCCCCGGGCCTTCCGGCCTTGCCGGGGAGCCTGGAA
AGCCTGGTATTCCCGGGCTCCCAGGCAGGGCTGGGGGTGTGGGAGAGGCAG
GAAGGCCAGGAGAGAGGGGAGAACGGGGAGAGAAAGGAGAACGTGGAGA
ACAGGGCAGAGATGGCCCTCCTGGACTCCCTGGAACCCCTGGGCCCCCCGG
ACCCCCTGGCCCCAAGGTGTCTGTGGATGAGCCAGGTCCTGGACTCTCTGGA
GAACAGGGACCCCCTGGACTCAAGGGTGCTAAGGGGGAGCCGGGCAGCAAT
GGTGACCAAGGTCCCAAAGGAGACAGGGGTGTGCCAGGCATCAAAGGAGA
CCGGGGAGAGCCTGGACCGAGGGGTCAGGACGGCAACCCGGGTCTACCAG
GAGAGCGTGGTATGGCTGGGCCTGAAGGGAAGCCGGGTCTGCAGGGTCCAA
GAGGCCCCCCTGGCCCAGTGGGTGGTCATGGAGACCCTGGACCACCTGGTG
CCCCGGGTCTTGCTGGCCCTGCAGGACCCCAAGGACCTTCTGGCCTGAAGG
GGGAGCCTGGAGAGACAGGACCTCCAGGACGGGGCCTGACTGGACCTACTG
GAGCTGTGGGACTTCCTGGACCCCCGGCCCTTCAGGCCTTGTGGGTCCACA
GGGGTCTCCAGGTTTGCCTGGACAAGTGGGGGAGACAGGGAAGCCGGGAG
CCCCAGGTCGAGATGGTGCCAGTGGAAAAGATGGAGACAGAGGGAGCCCTG
GTGTGCCAGGGTCACCAGGTCTGCCTGGCCCTGTCGGACCTAAAGGAGAAC
CTGGCCCCACGGGGGCCCCTGGACAGGCTGTGGTCGGGCTCCCTGGAGCAA
AGGGAGAGAAGGGAGCCCCTGGAGGCCTTGCTGGAGACCTGGTGGGTGAG
CCGGGAGCCAAAGGTGACCGAGGACTGCCAGGGCCGCGAGGCGAGAAGGG
TGAAGCTGGCCGTGCAGGGGAGCCCGGAGACCCTGGGGAAGATGGTCAGA
AAGGGGCTCCAGGACCCAAAGGTTTCAAGGGTGACCCAGGAGTCGGGGTCC
CGGGCTCCCCTGGGCCTCCTGGCCTCCAGGTGTGAAGGGAGATCTGGGCCT
CCCTGGCCTGCCCGGTGCTCCTGGTGTTGTTGGGTTCCCGGGTCAGACAGGC
CCTCGAGGAGAGATGGGTCAGCCAGGCCCTAGTGGAGAGCGGGGTCTGGCA

GGCCCCCCAGGGAGAGAAGGAATCCCAGGACCCCTGGGGCCACCTGGACCA

CCGGGGTCAGTGGGACCACCTGGGGCCTCTGGACTCAAAGGAGACAAGGG

AGACCCTGGAGTAGGGCTGCCTGGGCCCCGAGGCGAGCGTGGGGAGCCAGG

CATCCGGGGTGAAGATGGCCGCCCCGGCCAGGAGGGACCCCGAGGACTCAC

GGGGCCCCCTGGCAGCAGGGGAGAGCGTGGGGAGAAGGGTGATGTTGGGA

GTGCAGGACTAAAGGGTGACAAGGGAGACTCAGCTGTGATCCTGGGGCCTC

CAGGCCCACGGGGTGCCAAGGGGGACATGGGTGAACGAGGGCCTCGGGGC

TTGGATGGTGACAAAGGACCTCGGGGAGACAATGGGGACCCTGGTGACAAG

GGCAGCAAGGGAGAGCCTGGTGACAAGGGCTCAGCCGGGTTGCCAGGACT

GCGTGGACTCCTGGGACCCCAGGGTCAACCTGGTGCAGCAGGGATCCCTGG

TGACCCGGGATCCCCAGGAAAGGATGGAGTGCCTGGTATCCGAGGAGAAAA

AGGAGATGTTGGCTTCATGGGTCCCCGGGGCCTCAAGGGTGAACGGGGAGT

GAAGGGAGCCTGTGGCCTTGATGGAGAGAAGGGAGACAAGGGAGAAGCTG

GTCCCCCAGGCCGCCCCGGGCTGGCAGGACACAAAGGAGAGATGGGGGAG

CCTGGTGTGCCGGGCCAGTCGGGGGCCCCTGGCAAGGAGGGCCTGATCGGT

CCCAAGGGTGACCGAGGCTTTGACGGGCAGCCAGGCCCCAAGGGTGACCAG

GGCGAGAAAGGGGAGCGGGGAACCCCAGGAATTGGGGGCTTCCCAGGCCC

CAGTGGAAATGATGGCTCTGCTGGTCCCCCAGGGCCACCTGGCAGTGTTGGT

CCCAGAGGCCCCGAAGGACTTCAGGGCCAGAAGGGTGAGCGAGGTCCCCCC

GGAGAGAGAGTGGTGGGGGCTCCTGGGGTCCCTGGAGCTCCTGGCGAGAGA

GGGGAGCAGGGGCGGCCAGGGCCTGCCGGTCCTCGAGGCGAGAAGGGAGA

AGCTGCACTGACGGAGGATGACATCCGGGGCTTTGTGCGCCAAGAGATGAG

TCAGCACTGTGCCTGCCAGGGCCAGTTCATCGCATCTGGATCACGACCCCTC

CCTAGTTATGCTGCAGACACTGCCGGCTCCCAGCTCCATGCTGTGCCTGTGCT

CCGCGTCTCTCATGCAGAGGAGGAAGAGCGGGTACCCCCTGAGGATGATGA

GTACTCTGAATACTCCGAGTATTCTGTGGAGGAGTACCAGGACCCTGAAGCT

CCTTGGGATAGTGATGACCCCTGTTCCCTGCCACTGGATGAGGGCTCCTGCA

CTGCCTACACCCTGCGCTGGTACCATCGGGCTGTGACAGGCAGCACAGAGGC

CTGTCACCCTTTTGTCTATGGTGGCTGTGGAGGGAATGCCAACCGTTTTGGG

ACCCGTGAGGCCTGCGAGCGCCGCTGCCCACCCCGGGTGGTCCAGAGCCAG

GGGACAGGTACTGCCCAGGACTGA

Amino acid sequence of human type VII collagen (2944 AA) (SEQ ID NO: 2)

MTLRLLVAALCAGILAEAPRVRAQHRERVTCTRLYAADIVFLLDGSSSIGRSNFR
EVRSFLEGLVLPFSGAASAQGVRFATVQYSDDPRTEFGLDALGSGGDVIRAIREL
SYKGGNTRTGAAILHVADHVFLPQLARPGVPKVCILITDGKSQDLVDTAAQRLK
GQGVKLFAVGIKNADPEELKRVASQPTSDFFFFVNDFSILRTLLPLVSRRVCTTAG
GVPVTRPPDDSTSAPRDLVLSEPSSQSLRVQWTAASGPVTGYKVQYTPLTGLGQ
PLPSERQEVNVPAGETSVRLRGLRPLTEYQVTVIALYANSIGEAVSGTARTTALEG
PELTIQNTTAHSLLVAWRSVPGATGYRVTWRVLSGGPTQQQELGPGQGSVLLRD
LEPGTDYEVTVSTLFGRSVGPATSLMARTDASVEQTLRPVILGPTSILLSWNLVPE
ARGYRLEWRRETGLEPPQKVVLPSDVTRYQLDGLQPGTEYRLTLYTLLEGHEV
ATPATVVPTGPELPVSPVTDLQATELPGQRVRVSWSPVPGATQYRIIVRSTQGVE
RTLVLPGSQTAFDLDDVQAGLSYTVRVSARVGPREGSASVLTVRREPETPLAVPG
LRVVVSDATRVRVAWGPVPGASGFRISWSTGSGPESSQTLPPDSTATDITGLQPGT
TYQVAVSVLRGREEGPAAVIVARTDPLGPVRTVHVTQASSSSVTITWTRVPGATG
YRVSWHSAHGPEKSQLVSGEATVAELDGLEPDTEYTVHVRAHVAGVDGPPASV
VVRTAPEPVGRVSRLQILNASSDVLRITWVGVTGATAYRLAWGRSEGGPMRHQI
LPGNTDSAEIRGLEGGVSYSVRVTALVGDREGTPVSIVVTTPPEAPPALGTLHVV
QRGEHSLRLRWEPVPRAQGFLLHWQPEGGQEQSRVLGPELSSYHLDGLEPATQ
YRVRLSVLGPAGEGPSAEVTARTESPRVPSIELRVVDTSIDSVTLAWTPVSRASSY
ILSWRPLRGPGQEVPGSPQTLPGISSSQRVTGLEPGVSYIFSLTPVLDGVRGPEAS
VTQTPVCPRGLADVVFLPHATQDNAHRAEATRRVLERLVLALGPLGPQAVQVG
LLSYSHRPSPLFPLNGSHDLGIILQRIRDMPYMDPSGNNLGTAVVTAHRYMLAPD
APGRRQHVPGVMVLLVDEPLRGDIFSPIREAQASGLNVVMLGMAGADPEQLRR
LAPGMDSVQTFFAVDDGPSLDQAVSGLATALCQASFTTQPRPEPCPVYCPKGQK
GEPGEMGLRGQVGPPGDPGLPGRTGAPGPQGPPGSATAKGERGFPGADGRPGSP
GRAGNPGTPGAPGLKGSPGLPGRGDPGERGPRGPKGEPGAPGQVIGGEGPGLP
GRKGDPGPSGPPGPRGPLGDPGRGPPGLPGTAMKGDKGDRGERGPPGPGEGGI
APGEPGLPGLPGSPGPQGPVGPPGKKGEKGDSEDGAPGLPGQPGSPGEQGPRGP

PGAIGPKGDRGFPGPLGEAGEKGERGPPGPAGSRGLPGVAGRPGAKGPEGPPGP

TGRQGEKGEPGRPGDPAVVGPAVAGPKGEKGDVGPAGPRGATGVQGERGPPGL

VLPGDPGPKGDPGDRGPIGLTGRAGPPGDSGPPGEKGDPGRPGPPGPVGPRGRD

GEVGEKGDEGPPGDPGLPGKAGERGLRGAPGVRGPVGEKGDQGDPGEDGRNG

SPGSSGPKGDRGEPGPPGPPGRLVDTGPGAREKGEPGDRGQEGPRGPKGDPGLP

GAPGERGIEGFRGPPGPQGDPGVRGPAGEKGDRGPPGLDGRSGLDGKPGAAGP

SGPNGAAGKAGDPGRDGLPGLRGEQGLPGPSGPPGLPGKPGEDGKPGLNGKNG

EPGDPGEDGRKGEKGDSGASGREGRDGPKGERGAPGILGPQGPPGLPGPVGPP

GQGFPGVPGGTGPKGDRGETGSKGEQGLPGERGLRGEPGSVPNVDRLLETAGI

KASALREIVETWDESSGSFLPVPERRRGPKGDSGEQGPPGKEGPIGFPGERGLKG

DRGDPGPQGPPGLALGERGPPGPSGLAGEPGKPGIPGLPGRAGGVGEAGRPGER

GERGEKGERGEQGRDGPPGLPGTPGPPGPPGPKVSVDEPGPGLSGEQGPPGLKG

AKGEPGSNGDQGPKGDRGVPGIKGDRGEPGPRGQDGNPGLPGERGMAGPEGK

PGLQGPRGPPGPVGGHGDPGPPGAPGLAGPAGPQGPSGLKGEPGETGPPGRGLT

GPTGAVGLPGPPGPSGLVGPQGSPGLPGQVGETGKPGAPGRDGASGKDGDRGS

PGVPGSPGLPGPVGPKGEPGPTGAPGQAVVGLPGAKGEKGAPGGLAGDLVGEP

GAKGDRGLPGPRGEKGEAGRAGEPGDPGEDGQKGAPGPKGFKGDPGVGVPGS

PGPPGPPGVKGDLGLPGLPGAPGVVGFPGQTGPRGEMGQPGPSGERGLAGPPG

REGIPGPLGPPGPPGSVGPPGASGLKGDKGDPGVGLPGPRGERGEPGIRGEDGRP

GQEGPRGLTGPPGSRGERGEKGDVGSAGLKGDKGDSAVILGPPGPRGAKGDMG

ERGPRGLDGDKGPRGDNGDPGDKGSKGEPGDKGSAGLPGLRGLLGPQGQPGA

AGIPGDPGSPGKDGVPGIRGEKGDVGFMGPRGLKGERGVKGACGLDGEKGDK

GEAGPPGRPGLAGHKGEMGEPGVPGQSGAPGKEGLIGPKGDRGFDGQPGPKG

DQGEKGERGTPGIGGFPGPSGNDGSAGPPGPPGSVGPRGPEGLQGQKGERGPPG

ERVVGAPGVPGAPGERGEQGRPGPAGPRGEKGEAALTEDDIRGFVRQEMSQHC

ACQGQFIASGSRPLPSYAADTAGSQLHAVPVLRVSHAEEEERVPPEDDEYSEYSE

YSVEEYQDPEAPWDSDDPCSLPLDEGSCTAYTLRWYHRAVTGSTEACHPFVYG

GCGGNANRFGTREACERRCPPRVVQSQGTGTAQD*

In an embodiment, the COL7A 1 gene comprises or consists of a nucleic acid sequence having 70%, 80%, 85%, 90%, 95%, 96%, 97%, 98% or 99% or more sequence identity with the nucleic acid sequence of SEQ ID NO: 1. In a different embodiment, the COL7A1 gene comprises or consists of a nucleic acid sequence wherein 1 to 30, 1 to 20, 1 to 10, 1 to 5, 1 to 3, 1 to 2 or 1 base(s) is inserted, deleted, substituted, or added with respect to the nucleic acid sequence of SEQ ID NO: 1. In a further embodiment, the COL7A1 gene comprises or consists of the nucleic acid sequence of SEQ ID NO: 1.

In an embodiment, the type VII collagen comprises or consists of an amino acid sequence having 70%, 80%, 85%, 90%, 95%, 96%, 97%, 98% or 99% or more sequence identity with the amino acid sequence of SEQ ID NO: 2. In a different embodiment, the type VII collagen comprises or consists of an amino acid sequence wherein 1 to 30, 1 to 20, 1 to 10, 1 to 5, 1 to 3, 1 to 2 or 1 amino acid residue(s) is inserted, deleted, substituted, or added with respect to the amino acid sequence of SEQ ID NO: 2. In a further embodiment, the type VII collagen comprises or consists of the amino acid sequence of SEQ ID NO: 2.

In an embodiment, the COL7A1 gene comprises or consists of a nucleic acid sequence that encodes an amino acid sequence having 70%, 80%, 85%, 90%, 95%, 96%, 97%, 98% or 99% or more sequence identity with the amino acid sequence of SEQ ID NO: 2. In a different embodiment, the COL7A1 gene comprises or consists of a nucleic acid sequence that encodes an amino acid sequence wherein 1 to 30, 1 to 20, 1 to 10, 1 to 5, 1 to 3, 1 to 2 or 1 amino acid residue(s) is inserted, deleted, substituted, or added with respect to the amino acid sequence of SEQ ID NO: 2.

As used herein, the term "sequence identity" with respect to a nucleic acid sequence or an amino acid sequence means the proportion of bases or amino acid residues that match between two sequences that are optimally aligned (aligned to be maximally matched) over the entire region of the sequence to be compared. The sequence to be compared may have an insertion, an addition or a deletion (eg, a gap) in the optimal alignment of the two sequences. The sequence identity can be calculated using a program such as FASTA, BLAST, or CLUSTAL W provided in a public database (for example, DDBJ (http://www.ddbj.nig.ac.jp)). Alternatively, the sequence identity can be obtained using a commercially available sequence analysis software (for example, Vector NTI® software, GENETYX® ver. 12).

The cell may be genetically modified by any method. In an embodiment, the cell is genetically modified by genome editing such as the CRISPR system (eg, CRISPR/Cas9, CRISPR/Cpf1), TALEN, or ZFN. In a different embodiment, the cell is genetically modified with a viral vector such as a retroviral vector, lentiviral vector, adenoviral vector, or adeno-associated viral vector. In a further embodiment, the cell is genetically modified with CRISPR/Cas9. In a further embodiment, the cell is genetically modified with a retroviral vector or a lentiviral vector.

In genome editing, causing cleavage in the genome and introducing a donor vector comprising a sequence of interest into the cell can insert the sequence of interest into the cleavage site of the genome. The sequence to be inserted into the genome can be a COL7A1 gene or a sequence to be replaced with a portion containing a mutation in the COL7A1 gene (for example, a partial sequence of a COL7A1 gene). In addition to the sequence of interest, the donor vector may comprise a regulatory sequence such as a promoter or enhancer that controls the expression of the sequence of interest, or other elements such as a drug resistance gene for cell selection, and also may comprise, at both ends, sequences homogeneous to both ends of the insertion site of the genome. The donor vector can be introduced into a desired site as a result of non-homologous end binding or homologous recombination. As the donor vector, a plasmid, an adeno-associated viral vector, an integrase-deficient lentiviral vector, or any of other viral vectors can be used.

In the CRISPR system, an endonuclease such as Cas9 or Cas12 (eg, Cas12a (also called Cpf1), Cas12b, Cas12e) recognizes a specific base sequence, called PAM sequence, and the double strand of the target DNA is cleaved by the action of the endonuclease. When the endonuclease is Cas9, it cleaves about 3-4 bases upstream of the PAM sequence. Examples of endonucleases include Cas9 of S. pyogenes, S. aureus, N. meningitidis, S. thermophilus, or T. denticola, and Cpfl of L. bacterium ND2006 or Acidaminococcus sp. BV3L6. The PAM sequence varies depending on the endonuclease, and the PAM sequence of Cas9 in S. pyogenes is NGG, for example. A gRNA comprises a sequence of about 20 bases upstream of the PAM sequence (target sequence) or a sequence complementary thereto on the 5' end side, and plays a role of recruiting an endonuclease to the target sequence. The sequences other than the target sequence (or a sequence complementary thereto) of a gRNA can be appropriately determined by those skilled in the art depending on the endonuclease to be used. A gRNA may comprises a crRNA (CRISPR RNA), which comprises the target sequence or a sequence complementary thereto and is responsible for the sequence specificity of the gRNA, and a tracrRNA (Trans-activating crRNA), which contributes to the formation of a complex with Cas9 by forming a double strand. The crRNA and tracrRNA may exist as separate molecules. When the endonuclease is Cpfl, the crRNA alone functions as a gRNA. In the present specification, a gRNA comprising elements necessary for the function as a gRNA on a single strand may be particularly referred to as a sgRNA. The gRNA sequence can be determined by a tool available for target sequence selection and gRNA design, such as CRISPRdirect (https://crispr.dbcls.jp/).

A vector comprising a nucleic acid sequence encoding a gRNA and a nucleic acid sequence encoding an endonuclease may be introduced into and expressed in a cell, or a gRNA and an endonuclease protein that have been prepared extracellularly may be introduced into a cell. The endonuclease may include a nuclear localization signal. The nucleic acid sequence encoding a gRNA and the nucleic acid sequence encoding an endonuclease may be present on different vectors. Methods for introducing the vector, gRNA, and endonuclease into a cell include, but are not limited to, lipofection, electroporation, microinjection, calcium phosphate method, and DEAE-dextran method.

In an embodiment, a gRNA that can be used for the introduction of a COL7A1 gene into the genome comprises any of the sequences of SEQ ID NOs: 3 to 5 or a sequence complementary thereto.

In the case of viral vectors, a COL7A1 gene can be introduced into the genome of a cell when a retroviral vector or a lentiviral vector having integrase activity is used. Alternatively, an integrase-deficient retroviral or lentiviral vector may be used. Integrase-deficient vectors lack integrase activity, for example, due to a mutation in the integrase gene. When an integrase-deficient vector, or an adenoviral vector or an adeno-associated viral vector is used, the sequence incorporated into the vector is not usually introduced into the genome of a cell. For example, when a COL7A1 gene is incorporated into an integrase-deficient lentiviral vector or an adenoviral vector, type VII collagen is expressed from the COL7A1 gene of the vector existing in the cell (in the nucleus).

A viral vector comprises a sequence encoding a COL7A1 gene and may contain a regulatory sequence such as a promoter or enhancer that controls the expression of the COL7A1 gene and other elements such as a drug resistance gene for cell selection. A viral vector may be prepared by any method known in the art. For example, a retroviral or lentiviral vector can be prepared by introducing a viral vector plasmid comprising LTR sequences at both ends (5' LTR and 3' LTR), a packaging signal, and a sequence of interest into a packaging cell with one or more plasmid vectors expressing structural proteins of the virus, such as Gag, Pol, and Env, or into a packaging cell that expresses such structural proteins. Examples of packaging cells include, but are not limited to, 293T cells, 293 cells, HeLa cells, COS1 cells, and COS7 cells. The viral vector may be pseudotyped and may express an envelope protein such as the vesicular stomatitis virus G protein (VSV-G). The sequence of interest can be introduced into a target cell by infecting the target cell with a viral vector thus prepared.

In an embodiment, the viral vector is a lentiviral vector. Examples of lentiviral vectors include, but are not limited to, HIV (human immunodeficiency virus) (for example, HIV-1 and HIV-2), SIV (simian immunodeficiency virus), FIV (feline immunodeficiency virus), MVV (Maedi-Visna virus), EV1 (Maedi-Visna-like virus), EIAV (equine infectious anemia virus), and CAEV (caprine arthritis encephalitis virus). In an embodiment, the lentiviral vector is HIV.

As an example, a lentiviral vector can be prepared as follows. First, a viral vector plasmid encoding the viral genome, one or more plasmid vectors expressing Gag, Pol, and Rev (and optionally Tat), and one or more plasmid vectors expressing envelope proteins such as VSV-G are introduced into a packaging cell. The viral vector plasmid comprises LTR sequences at both ends (5' LTR and 3' LTR), a packaging signal, and a COL7A1 gene and a promoter that controls its expression (eg, CMV promoter, CAG promoter, EF1α promoter, PGK promoter, or hCEF promoter). The 5' LTR functions as a promoter that induces transcription of the viral RNA genome, but may be replaced with a different promoter, such as CMV promoter, to enhance the expression of the RNA genome. Within the cell, the viral RNA genome is transcribed from the vector plasmid and packaged to form a viral core. The viral core is transported to the cell membrane of the packaging cell, encapsulated in the cell membrane, and released as a viral particle from the packaging cell. The released virus particle can be recovered from the culture supernatant of the packaging cell. For example, the virus particle can be recovered by any of conventional purification methods such as centrifugation, filter filtration, and column purification. A lentiviral vector can also be prepared by using a kit such as Lentiviral High Titer Packaging Mix, Lenti-X™ Packaging Single Shots (Takara Bio Inc.), and ViraSafe™ Lentivirus Complete Expression System (Cell Biolabs Inc.). An adeno-associated viral vector can be prepared by using a kit such as AAVpro® Helper Free System (Takara Bio Inc.).

In one aspect, the present disclosure provides a plasmid for producing a lentiviral vector comprising an EF1α promoter and a COL7A1 gene located downstream of the EF1α promoter. In a further aspect, the disclosure provides a lentiviral vector comprising an EF1α promoter and a COL7A1 gene located downstream of the EF1α promoter.

A representative sequence of the EF1α promoter is shown in SEQ ID NO:6.

EF1α promoter (SEQ ID NO: 6)

```
GTGAGGCTCCGGTGCCCGTCAGTGGGCAGAGCGCACATCGCCCACAGTCCC
CGAGAAGTTGGGGGGAGGGGTCGGCAATTGAACCGGTGCCTAGAGAAGGTG
GCGCGGGGTAAACTGGGAAAGTGATGTCGTGTACTGGCTCCGCCTTTTTCCC
GAGGGTGGGGGAGAACCGTATATAAGTGCAGTAGTCGCCGTGAACGTTCTTT
TTCGCAACGGGTTTGCCGCCAGAACACAGGTAAGTGCCGTGTGTGGTTCCCG
CGGGCCTGGCCTCTTTACGGGTTATGGCCCTTGCGTGCCTTGAATTACTTCCA
CGCCCCTGGCTGCAGTACGTGATTCTTGATCCCGAGCTTCGGGTTGGAAGTG
GGTGGGAGAGTTCGAGGCCTTGCGCTTAAGGAGCCCCTTCGCCTCGTGCTTG
AGTTGAGGCCTGGCTTGGGCGCTGGGGCCGCCGCGTGCGAATCTGGTGGCA
CCTTCGCGCCTGTCTCGCTGCTTTCGATAAGTCTCTAGCCATTTAAAATTTTTG
ATGACCTGCTGCGACGCTTTTTTTCTGGCAAGATAGTCTTGTAAATGCGGGCC
```

AAGATCTGCACACTGGTATTTCGGTTTTTGGGGCCGCGGGCGGCGACGGGGC

CCGTGCGTCCCAGCGCACATGTTCGGCGAGGCGGGGCCTGCGAGCGCGGCC

ACCGAGAATCGGACGGGGGTAGTCTCAAGCTGGCCGGCCTGCTCTGGTGCCT

GGCTCGCGCCGCCGTGTATCGCCCCGCCCTGGGCGGCAAGGCTGGCCCGGT

CGGCACCAGTTGCGTGAGCGGAAAGATGGCCGCTTCCCGGCCCTGCTGCAG

GGAGCTCAAAATGGAGGACGCGGCGCTCGGGAGAGCGGGCGGGTGAGTCA

CCCACACAAAGGAAAAGGGCCTTTCCGTCCTCAGCCGTCGCTTCATGTGACT

CCACGGAGTACCGGGCGCCGTCCAGGCACCTCGATTAGTTCTCGAGCTTTTG

GAGTACGTCGTCTTTAGGTTGGGGGGAGGGGTTTTATGCGATGGAGTTTCCC

CACACTGAGTGGGTGGAGACTGAAGTTAGGCCAGCTTGGCACTTGATGTAAT

TCTCCTTGGAATTTGCCCTTTTTGAGTTTGGATCTTGGTTCATTCTCAAGCCTC

AGACAGTGGTTCAAAGTTTTTTTCTTCCATTTCAGGTGTCGTGAA

In an embodiment, the EF1α promoter comprises or consists of a nucleic acid sequence having 70%, 80%, 85%, 90%, 95%, 96%, 97%, 98% or 99% or more sequence identity with the nucleic acid sequence of SEQ ID NO: 6. In a different embodiment, the EF1α promoter comprises or consists of a nucleic acid sequence wherein 1 to 30, 1 to 20, 1 to 10, 1 to 5, 1 to 3, 1 to 2 or 1 base(s) is inserted, deleted, substituted, or added with respect to the nucleic acid sequence of SEQ ID NO: 6. In a further embodiment, the EF1α promoter comprises or consists of the nucleic acid sequence of SEQ ID NO: 6.

A cell into which a sequence of interest has been introduced can be detected by Southern blotting or PCR. The sequence of interest may only be introduced into at least one of the alleles.

In one embodiment, a cell sheet is transplanted onto a lesion of a patient with dystrophic epidermolysis bullosa. In a preferred embodiment, the cell sheet is transplanted onto a part of the skin that lacks epidermis. Specific exmaples of the part of the skin that lacks epidermis include a surface of an ulcer of the skin. Alternatively, the cell sheet may be transplanted at a place where the epidermis is detached from the dermis. In one embodiment, the cell sheet is transplanted by sticking it to the lesion of the skin. In this case, it is preferable to stick the cell sheet to the lesion so that the cell sheet attaches to the dermis.

Accordingly, in an aspect, the present disclosure provides a method for treating dystrophic epidermolysis bullosa comprising transplanting the cell sheet of the present disclosure to the skin of a patient with dystrophic epidermolysis bullosa.

In an aspect, the present disclosure provides a composition comprising the cell sheet of the present disclosure. The composition can be used for treating dystrophic epidermolysis bullosa. The composition may comprise a pharmaceutically acceptable carrier and/or an additive in addition to the cell sheet. Examples of pharmaceutically acceptable carriers include water, medium, saline, infusion containing glucose, D-sorbitol, D-mannitol or others, and phosphate buffered saline (PBS). Examples of additives include solubilizers, stabilizers, and preservatives. The composition may be frozen and may contain a cryoprotectant such as DMSO, glycerol, polyvinylpyrrolidone, polyethylene glycol, dextran, or sucrose.

Embodiments of a method for producing a cell sheet are provided below. Fig. 1 shows an outline of the method for producing a cell sheet. Each process is outlined as follows.

1. A body fluid within a blister of a patient with dystrophic epidermolysis bullosa is collected.
2. At least a portion of the collected body fluid is inoculated into a medium and the medium is cultured.
3. Cells adhering to the bottom of a culture vessel are obtained. Then, if necessary, these cells are passaged.
4. A type VII collagen gene is introduced into the obtained cells.
5. The cells into which the type VII collagen gene is introduced are cultured and, if necessary, passaged.
6. The cells into which the type VII collagen gene is introduced are seeded on a culture vessel.
7. The cultured cells are taken out from the culture vessel as a cell sheet.

1. Collection of a blister fluid

The explanation of the method of collecting a body fluid within a blister is omitted here since it has been described above.

2. Inoculation of the blister fluid and culturing

At least a portion of the collected body fluid is inoculated into a medium and the medium is cultured. Said at least a portion of a body fluid comprises one component of the body fluid. Said one component may be a precipitate after the centrifugation of the body fluid. Said one component may also be a fraction obtained by using any of devices capable of isolating specific cells, such as magnetic beads or flow cytometers. As described above, the body fluid within a blister, one component of the body fluid, or cells obtained from the body fluid are preferably inoculated into a medium without enzymatic treatment. The explanation of the methods of inoculating and culturing are omitted here since they have been described above.

3. Passage of the adherent cells

After inoculating and culturing, cells adhering to the bottom of the culture vessel are obtained. Then, the cells are passaged as necessary and repeatedly as appropriate. This provides the number of cells needed for gene transfer. During the course of passaging, the cells can be frozen and preserved, if necessary. Cryopreservation of the blister-derived cells can be performed by general work processes using common cell cryopreservation solutions. The cell cryopreservation solution may be any solution that can be used for cryopreservation of animal cells, such as CELLBANKER® 1 (ZENOGEN PHARMA CO., LTD.), STEM-CELLBANKER GMP grade (ZENOGEN PHARMA CO., LTD.), STEM-CELLBANKER DMSO Free GMP grade (ZENOGEN PHARMA CO., LTD.), HSC-BANKER® GMP grade (ZENOGEN PHARMA CO., LTD.), or a medium supplemented with 5-10% DMSO. Among these, cryopreservation solutions for stem cells such as STEM-CELLBANKER GMP grade and HSC-BANKER® GMP grade are preferably used. The cell cryopreservation solution is preferably serum-free.

4. Gene transfer

Into the passaged cells, a type VII collagen gene is introduced. The explanation of the method of gene transfer is omitted here since it has been described above. In this process, the type VII collagen gene is preferably introduced into the blister-derived cells with a lentiviral vector.

5. Passage of the gene-transferred cells

The cells into which the type VII collagen gene is introduced are cultured, and passaged as necessary and repeatedly as appropriate. This provides the number of cells needed to produce the cell sheet. During the course of passaging, the cells can be frozen and preserved, if necessary. The cryopreservation solution described above can be used for cryopreservation of the cells.

6. Seeding of the gene-transferred cells

The blister-derived cells into which the type VII collagen gene is introduced are seeded in a culture vessel. Preferably, the cells are seeded in a culture vessel to be overconfluent. Specifcially, the cells are seeded in a culture vessel preferably to be 150% or more confluent, more preferably to be 300% or more confluent, and even more preferably to be 600% or more confluent. Seeding the cells in such confluency provides a cell sheet with a layered structure suitable for the sheet transplantation.

The blister-derived cells into which the type VII collagen gene is introduced are seeded in a culture vessel preferably at a cell density of $1 \times 10^4$ cells/cm$^2$ or higher, more preferably at a cell density of $5 \times 10^4$ cells/cm$^2$ or higher, even more preferably at a cell density of $1 \times 10^5$ cells/cm$^2$ or higher. Seeding the cells at such a density provides a cell sheet having an optional number of cell layers, which has good mechanical strength and allows oxygen to be available to the cells inside the cell sheet.

The culture vessel can be any of ordinary culture vessels with polystyrene surfaces. In other words, temperature-responsive culture vessels are not necessarily required. The inventors have found that the blister-derived cells can be detached from the bottom of the culture vessel without the use of temperature-responsive culture vessels. Of course, the blister-derived cells may be cultured in a temperature-responsive culture vessel. Examples of temperature-responsive culture vessels include a culture vessel whose surface is treated with a temperature-responsive polymer. The temperature-responsive polymer changes from hydrophobic state to hydrophilic state at a specific temperature, e.g., 32°C. Thus, for example, at 37°C, the cells adhere to the temperature-responsive polymer, and when the temperature is lowered to, for example, 20°C, the cells become unable to adhere to the temperature-responsive polymer and detach from the bottom of the culture vessel.

7. Collection of the cell sheet

The cultured cells are taken out from the culture vessel as a cell sheet. The inventors have found that the blister-derived cells can be detached as a cell sheet from the culture vessel without treatment with trypsin, dispase, or any other enzyme. The cell sheet comprising the blister-derived cells can be detached and picked up with tweezers alone, even from the bottom of a normal culture vessel that is not a temperature-responsive culture vessel. It is, however, desirable to use a support to maintain the shape of the cell sheet. Of course, a temperature-responsive culture vessel may be used to collect the cell sheet and the cell sheet may be detached and picked up by lowering the temperature of the vessel. In this case, it is also desirable to use a support to maintain the shape of the cell sheet. The support refers to a membrane or sheet material to which the cell sheet is attached to reinforce the strength of the cell sheet, such as a nonwoven fabric, hydrophilic PVDF membrane, nitrocellulose membrane, gauze, or a membrane composed of bioabsorbable material.

Exemplary embodiments of the present invention are described below.

[1] A cell sheet for transplantation onto a skin of a patient with dystrophic epidermolysis bullosa, comprising cells which are derived from a body fluid within a blister of a patient with dystrophic epidermolysis bullosa and into which a type VII collagen gene is introduced.

[2] The cell sheet according to item 1, wherein the type VII collagen gene is introduced into a genome of the cells.

[3] The cell sheet according to item 1 or 2, wherein

a type VII collagen gene expression cassette is introduced into the cells, and
the type VII collagen gene expression cassette comprises an EF1$\alpha$ promoter and the type VII collagen gene located downstream of the EF1$\alpha$ promoter.

[4] The cell sheet according to any one of items 1 to 3, wherein the type VII collagen gene expression cassette is introduced into a genome of the cells.

[5] The cell sheet according to any one of items 1 to 4, wherein the type VII collagen gene comprises a nucleic acid sequence having 70%, 80%, 85%, 90%, 95%, 96%, 97%, 98% or 99% or more sequence identity with the nucleic acid sequence of SEQ ID NO: 1, or a nucleic acid sequence that encodes an amino acid sequence having 70%, 80%, 85%, 90%, 95%, 96%, 97%, 98% or 99% or more sequence identity with the amino acid sequence of SEQ ID NO: 2.

[6] The cell sheet according to any one of items 1 to 5, wherein the cells overlap each other in a thickness direction of the sheet.

[7] The cell sheet according to any one of items 1 to 6, wherein, in a longitudinal section of the cell sheet, the cell sheet has a higher cell density near a surface of the sheet than in a central portion of the sheet.

[8] The cell sheet according to any one of items 1 to 7, wherein the cell sheet has a thickness of 5 to 50 $\mu$m.

[9] The cell sheet according to any one of items 1 to 8, wherein the cell sheet is transplanted onto a lesion.

[10] The cell sheet according to any one of items 1 to 9, wherein the cell sheet is transplanted onto a part of the skin that lacks epidermis.

[11] A composition for treating dystrophic epidermolysis bullosa, comprising the cell sheet according to any one of items 1 to 10.

[12] The composition according to item 11, wherein the cell sheet is transplanted onto a lesion.

[13] The composition according to item 10 or 11, wherein the cell sheet is transplanted onto a part of the skin that lacks epidermis.

[14] A method for treating dystrophic epidermolysis bullosa, comprising transplanting the cell sheet according to any one of items 1 to 10 onto the skin of a patient with dystrophic epidermolysis bullosa.

[15] The method according to item 14, wherein the cell sheet is transplanted onto a lesion.

[16] The method according to item 14 or 15, wherein the cell sheet is transplanted onto a part of the skin that lacks epidermis.

[17] A method for producing a cell sheet for transplantation onto a skin of a patient with dystrophic epidermolysis bullosa, comprising the steps of:

inoculating at least a portion of a body fluid within a blister of a patient with dystrophic epidermolysis bullosa into a medium,
obtaining cells adhering to a bottom of a culture vessel,

introducing a type VII collagen gene into the cells, and

culturing the cells comprising the introduced type VII collagen gene, and

taking out the cultured cells from a culture vessel as a cell sheet.

[18] The method for producing a cell sheet according to item 17, comprising inoculating cells obtained from the body fluid within the blister into the medium without enzymatic treatment.

[19] The method for producing a cell sheet according to item 17 or 18, wherein the type VII collagen gene is introduced into the cells with a lentiviral vector.

[20] The method for producing a cell sheet according to item 19, wherein

the lentiviral vector comprises a type VII collagen gene expression cassette, and

the type VII collagen gene expression cassette comprises an EF1$\alpha$ promoter and the type VII collagen gene located downstream of the EF1$\alpha$ promoter.

[21] The method for producing a cell sheet according to any one of items 17 to 20, wherein the type VII collagen gene comprises a nucleic acid sequence having 70%, 80%, 85%, 90%, 95%, 96%, 97%, 98% or 99% or more sequence identity with the nucleic acid sequence of SEQ ID NO: 1, or a nucleic acid sequence that encodes an amino acid sequence having 70%, 80%, 85%, 90%, 95%, 96%, 97%, 98% or 99% or more sequence identity with the amino acid sequence of SEQ ID NO: 2.

[22] The method for producing a cell sheet according to any one of items 17 to 21, further comprising the step of seeding the cells comprising the introduced type VII collagen gene to be overconfluent.

[23] The method for producing a cell sheet according to any one of items 17 to 22, further comprising the step of seeding the cells comprising the introduced type VII collagen gene into a culture vessel at a cell density of $1 \times 10^4$ cells/cm$^2$ or higher.

[23] The method for producing a cell sheet according to any one of items 17 to 23, comprising taking out the cultured cells from the culture vessel as the cell sheet without enzymatic treatment.

The present invention is described in more detail with reference to the examples hereinafter, but not limited to the embodiments described below.

Examples

1. Collection of blister-derived cells

Blister fluid from a patient with dystrophic epidermolysis bullosa was collected and centrifuged at 300 g for 5 minutes. The resulting precipitate was suspended in a medium (Mesenchymal Stem Cell Growth Medium 2 (PromoCell, C-28009) supplemented with penicillin and streptomycin to a final concentration of 100 unit/mL and 100 μg/mL, respectively), and seeded in a 6-well plate coated with collagen I and cultured at 37°C and 5% $CO_2$ to obtain adherent cells. The duration time from collection of the blister fluid to inoculation into the medium varied from patient to patient, but was within the range of 18 minutes to 1 hour. Thereafter, medium exchange and passaging were carried out appropriately until the cells proliferated to the desired cell number (culture progress up to 20 days after initiation of culture is shown in Fig. 2). Cells obtained by such a method are hereinafter also referred to as "blister-derived cells". Cells at the 3rd passage were used for the following surface marker analysis and differentiation induction experiment, and cells at the 3rd to 4th passage were used for gene transfer. It was confirmed that blister-derived cells obtained with a mixed medium of equal volume of Mesenchymal Stem Cell Growth Medium 2 (PromoCell, C-28009) and MSCGM Mesenchymal Stem Cell Growth Medium (Lonza, PT-3001) or Cellartis MSC Xeno-Free Culture Medium (Takara Bio, Y50200) were equivalent to the blister-derived cells obtained with the above medium. As a preliminary study, we investigated the correlation between the duration time from collecting the blister fluid from the patient's blister to inoculating it in the medium and the number of colonies formed on the plate after culturing, and found that a large number of colonies were obtained when the blister fluid was inoculated within 1 hour after the collection of the blister fluid, and very few or almost no colony was obtained when the blister fluid was inoculated more than 3 hours after the collection.

2. Characterization of blister-derived cells

a) Surface marker analysis (FACS)

The blister-derived cells obtained in the section 1 above and human bone marrow-derived mesenchymal stem cells (hereinafter also referred to as BM-MSCs) [purchased from PromoCell (Heidelberg, Germany) or Lonza (Basel, Switzerland)] were subjected to surface marker analysis according to the following procedure. The cells were removed from the plate with Accutase-Solution (PromoCell, C-41310), and after washed with medium, 100,000 cells each were put into two tubes based on cell count results. The cells were washed once with Flow Cytometry Staining Buffer (1X) (R&D Systems, FC001), and resuspended with 100 $\mu$l of Flow Cytometry Staining Buffer (1X). To block Fc receptors, 5 $\mu$l of Human TruStain FcX™ (BioLegend, 422301) was added and allowed to react on ice for 10 minutes. To one of the tubes, 10 $\mu$l each of CD73-CFS Mouse IgG2B, CD90-APC Mouse IgG2A, and Negative Marker Cocktail (CD45-PE Mouse IgG1, CD34-PE Mouse IgG1, CD11b-PE Mouse IgG2B, CD79A-PE Mouse IgG1, HLA-DR-PE Mouse IgG1), which were contained in Human Mesenchymal Stem Cell Verification Flow Kit (R&D Systems, FMC020), and further 5 $\mu$l of Brilliant Violet 421™ anti-human CD105 Antibody (BioLegend, 323219) were added, and allowed to react for 30 minutes at room temperature in the dark. Since the other tube was used as a negative control, the same amount of each isotype control antibody was added and allowed to react for 30 minutes at room temperature in the dark. The cells were washed once with 2 ml of Flow Cytometry Staining Buffer (1$\times$), resuspended in 300 $\mu$l of Flow Cytometry Staining Buffer (1$\times$), and analyzed by BD FACSAria (BD). In addition, whether CD31 was expressed in blister-derived cells and human BM-MSCs was analyzed by FACS analysis according to the following procedure. The cells were removed from the plate with Accutase-Solution (PromoCell, C-41310), and after washed with medium, 100,000 cells each were put into two tubes based on cell count results. The cells were washed once with 2% FBS-containing PBS and resuspended in 100 $\mu$l of 2% FBS-containing PBS. To block Fc receptors, 5 $\mu$l of Human TruStain FcX™ (BioLegend, 422301) was added and allowed to react on ice for 10 minutes. To one of the tubes, 5 $\mu$l of APC anti-human CD31 Antibody (BioLegend, 303116) (0.4 $\mu$g of protein) was added and allowed to react for 60 minutes on ice in the dark. Since the other tube was used as a negative control, 2 $\mu$l of APC Mouse IgG1, $\kappa$ Isotype Ctrl Antibody (BioLegend, 400120) (0.4 $\mu$g of protein) was added and allowed to react for 60 minutes on ice in the dark. The cells were washed once with 2 ml of 2% FBS-containing PBS, resuspended in 300 $\mu$l of 2% FBS-containing PBS, and analyzed by BD FACSAria (BD).

FACS analysis showed that both the blister-derived cells and BM-MSCs were positive for CD73, CD105 and CD90 and negative for CD45, CD34, CD11b, CD79A, HLA-DR and CD31 (Fig. 3).

b) Induction of differentiation (osteoblasts, adipocytes and chondrocytes)

The blister-derived cells obtained in the section 1 above and BM-MSCs were induced to differentiate into osteoblasts, adipocytes and chondrocytes under the following conditions.

Induction of differentiation into osteoblasts:

Cells were cultured in a medium containing 0.1 $\mu$M Dexamethasone, 0.2 mM Ascorbic acid 2-phosphate, 10 mM Glycerol 2-phosphate (all values were final concentrations) at 37°C and 5% $CO_2$ for 3 weeks (the medium was changed twice a week) to induce differentiation into osteoblasts. The cells were stained with Alkaline phosphatase (ALP) using TRACP & ALP Assay Kit (Takara Bio Inc., MK301) according to the product manual.

Induction of differentiation into adipocytes:

Cells were cultured in a medium containing 1 $\mu$M Dexamethasone, 0.5 mM 3-Isobutyl-1-methylanxthine (IBMX), 10 $\mu$g/mL Insulin, and 100 $\mu$M Indomethacin (all values were final concentrations) at 37°C and 5% $CO_2$ for 3 weeks (the medium was changed twice a week) to induce differentiation into adipocytes. The cells were stained with Oil Red O using a LipiD Assay kit (Cosmo Bio, AK09F) according to the product manual.

Induction of differentiation into chondrocytes:

A chondrogenic differentiation medium (incomplete medium) was prepared by mixing the components of Human Mesenchymal Stem Cell (hMSC) Chondrogenic Differentiation Medium Bullet Kit (tm) (Lonza, PT-3003) according to the instructions. Recombinant Human TGF-beta 3 Protein (R&D Systems, 243-B3) was added to the medium to a final concentration of 10 ng/ml to prepare a chondrogenic differentiation medium (complete medium) each time it was used. After the third passage cells were detached with Accutase-Solution PromoCell, C-41310) and washed with a medium, 250,000 cells each were put into 15 ml polypropylene conical tubes based on cell count results. The cells were washed twice with the chondrogenic differentiation medium (incomplete medium), the supernatant was removed, and the cells were suspended in 500 $\mu$l of the chondrogenic differentiation medium (complete medium). The cells were centrifuged at 150 g for 5 minutes to form a cell pellet, and after the lid of the tube was unscrewed, the tube was placed in a $CO_2$ incubator (37°C, 5% $CO_2$), and then the medium (complete medium) was changed every 2 to 3 days. After 3 weeks, the pellet was removed and fixed with 4% paraformaldehyde, frozen sections were prepared, and chondrocyte-derived proteoglycans were stained with Alcian Blue. As a positive control, human bone marrow-derived mesenchymal stem cells were also subjected to the same differentiation-inducing operation.

The results of the differentiation induction experiments are shown in Fig. 4. BM-MSCs were positive in all of the ALP staining, Oil Red O staining, and Alcian Blue staining. The blister-derived cells were positive in the ALP staining (but staining intensity was lower than that of BM-MSCs), positive in the Oil Red O staining (but staining intensity was lower than that of BM-MSCs), and positive in the Alcian blue staining (but staining intensity was lower than that of BM-MSCs).

c) Evaluation of ability to express and secrete type VII collagen

How much blister-derived cells expressed and secreted type VII collagen was examined by Western blotting. In this experiment, blister-derived cells were obtained from a patient who expressed type VII collagen, but whose expressed type VII collagen was thought to have little function due to amino acid mutation. First, human epidermal keratinocytes (hereinafter referred to as KC), human skin fibroblasts (hereinafter referred to as FB), human bone marrow-derived mesenchymal stem cells (hereinafter referred to as MSC), and blister-derived cells of an epidermolysis bullosa patient (hereinafter BFC; also referred to as blister fluid cells) were cultured in the media shown in Table 1 below.

Table 1

| Cells | Medium |
|---|---|
| KC: Human Epidermal Keratinocyte (NB)* (KURABOU, KK-4009) *(NB means "New Born") | Humedia-KG2 (KURABO, KK-2150S) |
| FB: Normal Human Dermal Fibroblasts - Adult (Lonza, CC-2511) | Fibroblast Growth Medium-2 BulletKit (Lonza, CC-3132) |
| MSC: hMSC - Human Mesenchymal Stem Cells (Lonza, PT-2501) | MSCGM Mesenchymal Stem Cell Growth Medium (Lonza, PT-3001) |
| BFC: blister-derived cells (collected from blister fluid of epidermolysis bullosa patient) | Mixed medium of equal volume of Mesenchymal Stem Cell Growth Medium 2 (PromoCell, C-28009) and MSCGM Mesenchymal Stem Cell Growth Medium (Lonza, PT-3001) |

When the cells reached 90-95% confluency, the cells were washed with D-PBS(-). Each medium (no supplement added) containing ascorbic acid (Nacalai Tesque, 13048-42, final concentration of 50 ug/ml) and a protease inhibitor cocktail (SIGMA, P1860-1ML, 1/400 dilution) was added to the cells, and the cells were cultured in a $CO_2$ incubator for 24 hours. After the culture, the medium was concentrated using a methanol-chloroform precipitation method. A cell lysate was also prepared from the cells using RIPA buffer (Nacalai Tesque, 08714-04). Each lysate was corrected based on protein concentration, and a sample for electrophoresis was prepared using LDS sample buffer and sample reducing agent (Invitrogen, NP0007 and NP0009, respectively). After electrophoresis with 3-8% NuPAGE gel (Invitrogen, EA0375-BOX), the gel was transferred to a PVDF membrane (Millipore, IPVH07850), and the membrane was reacted with Anti-Col7 (Atlas, HPA042420) as a primary antibody and Anti-Rabbit IgG-HRP (GE healthcare, NA9340-1ML) as a secondary antibody. Then, the bands were detected using Chemi-lumi-one ultra (Nacalai Tesque, 11644-40) and Chemi DOC (BioRad, 17001402JA), and analyzed and quantified with Image Lab software (BioRad, 1709690). The western blotting was also performed on the concentrated medium in the same manner to quantify the concentration of type VII collagen.

The results are shown in Fig. 5. As shown in Fig. 5 (top), the western blotting of the cell lysate revealed that the expression level of COL7A in the blister-derived cells was higher than that of the skin fibroblasts and bone marrow-derived mesenchymal stem cells, and at a level comparable to that of the epidermal keratinocytes. In addition, as shown in Fig. 5 (bottom), the western blotting on the concentrate of the medium in which the cells were cultured revealed that the amount of COL7A secreted from the blister-derived cells was higher than that from any other cells. From these results, blister-derived cells were considered to be optimal cells to express type VII collagen by gene transfer.

3. Design of genome editing

Three types of sgRNAs were prepared in order to select a site with good cleavage efficiency by CRISPR-Cas9 system in AAVS1 (Adeno-associated virus integration site 1) region in the human genome. The AAVS1 region is a safe region that is not easily affected by gene transfer (safe harbor). Since the CRISPR-Cas9 system recognized the base sequence of "NGG" and cleaved 3 bases upstream of the sequence, regions each having "GG" at the end were selected and sgRNAs each containing a target sequence of 20 bases upstream of "NGG" were designed (sgAAVS1-#1 to #3) (Fig. 6, top; Table 2).

Table 2

| sgRNA | Target sequence | SEQ ID NO. |
|---|---|---|
| sgAAVS1-#1 | ACCCCACAGTGGGGCCACTA | 3 |
| sgAAVS1-#2 | GTCACCAATCCTGTCCCTAG | 4 |
| sgAAVS1-#3 | GGGGCCACTAGGGACAGGAT | 5 |

An oligonucleotide consisting of a sequence of any one of SEQ ID NOs: 3 to 5 was annealed with its complementary strand and cloned into the Bbs1 site of eSpCas9 (1.1) (Addgene plasmid # 71814) to prepare a plasmid that expressed Cas9 protein and sgRNA (eSpCas9(1.1)-sgAAVS1-#1, eSpCas9(1. 1)-sgAAVS 1-#2, or eSpCas9(1.1)-sgAAVS1-#3). This plasmid (2.5 μg) was introduced into HEK293 cells (human fetal kidney cell line) seeded in 6-well dishes by Lipofectamin 3000 (Thermo Fisher Scientific). Forty-eight hours after transfection, genomic DNA was extracted from the cells and the region containing the target site was amplified by PCR. The PCR amplified fragments were subjected to heat treatment to be single chains, and they were annealed by slow cooling and then treated with a mismatch site-specific endonuclease. The resulting product was fractionated by electrophoresis, the degree of insertion or deletion mutation introduced by the genome cleavage was measured from the density of the band, and the genome editing efficiency was calculated by the following formula (In the formula, "a" indicates the concentration of the band that was not digested, and "b" and "c" indicate the concentrations of the cleaved bands.).

$$\mathrm{Indel}\,(\%) = 100 \times (1 - \sqrt{(1 - f\mathrm{cut})}), \quad f_{\mathrm{cut}} = (b+c)/(a+b+c)$$

All sgRNAs of sgAAVS1-#1 to #3 produced a short DNA fragment different from the control, confirming that double-strand break occurred (Fig. 6, bottom). In the following experiments, sgAAVS1-#3, which had the highest cleavage efficiency, was used.

4. Introduction of COL7A1 gene into blister-derived cells

For introduction of a COL7A1 gene into the AAVS1 region, a plasmid expressing a COL7A1 gene under the control of a CAG promoter was designed (Fig. 7). COL7A1 cDNA was obtained from Flexi ORF sequence-verified clone (Promega, Madison, WI, USA). The COL7A1 cDNA was subcloned into the pENTR1A plasmid (Thermo Fisher Scientific, A10462) to prepare pENTR1A-COL7A1. COL7A1 cDNA was transferred from pENTR1A-COL7A1 to pAAVS1-P-CAG-DEST (Addgene plasmid # 80490) by Gateway reaction using LR recombinase (Thermo Fisher Scientific) to obtain a donor plasmid pAAVS1-P-CAG-COL7A1.

The blister-derived cells obtained in the section 1 above were suspended in a special buffer for the Neon transfection system (Thermo Fisher Scientific), and mixed with the Cas9-sgRNA expression plasmid (eSpCas9(1.1)-sgAAVS1-#3) and the donor plasmid (pAAVS1-P-CAG-COL7A1) as follows.

Table 3

| Blister-derived cells | $1 \times 10^5$ cells |
|---|---|
| eSpCas9(1.1)-sgAAVS1-#3 | 0.5 μg |
| pAAVS1-P-CAG-COL7A1 | 0.25 μg |

Using the Neon transfection system, the plasmid was introduced into blister-derived cells by electroporation under the conditions of 1200 V, 20 ms, and 2 pulses, and the cells were seeded on a 6-well plate and cultured. The medium was a mixed medium of equal volume of Mesenchymal Stem Cell Growth Medium 2 (PromoCell, C-28009) and MSCGM Mesenchymal Stem Cell Growth Medium (Lonza, PT-3001). Forty-eight hours after transfection, puromycin was added to a final concentration of 0.5 μg/mL, and the cells were cultured for about 2 weeks. The selected cells were treated as described in "5. Transplantation of genetically modified blister-derived cells into mice".

We also constructed a donor plasmid that expressed a COL7A1 gene under the control of a PGK promoter, and introduced this plasmid into various cells, including blister-derived cells. Then, the expression level and secretion level of COL7A1 in the modified cells were evaluated by Western blotting in the same manner as in "c) Evaluation of ability to express and secrete type VII collagen" in "2. Characterization of blister-derived cells" above. In this experiment, blister-derived cells were obtained from a patient who did not express type VII collagen. The results are shown in Fig. 8. As shown in Fig. 8 (top), more type VII collagen was detected in the blister-derived cell lysate than in the fibroblast lysate. Similarly, as shown in Fig. 8 (bottom), more type VII collagen was detected in the medium in which blister-derived cells were cultured than in the medium in which fibroblasts were cultured. From these results, it was found that when a COL7A1 gene was introduced into cells with CRISPR-Cas9, blister-derived cells expressed and secreted more type VII collagen than fibroblasts. (It was confirmed by immunostaining and Western blotting of culture supernatant that cells expressing and secreting type VII collagen were obtained even when blister-derived cells were genetically modified with a COL7A1 gene donor plasmid using an EF1α promoter.)

5. Infection efficiency of lentivirus to blister-derived cells

The infection efficiency of lentivirus to blister-derived cells was analyzed. The cells used in this experiment and the medium used to culture the cells are shown in Table 4 below.

Table 4

| Cells | Medium |
|---|---|
| BFC: blister-derived cells (collected from blister fluid of epidermolysis bullosa patient) | Mixed medium of equal volume of Mesenchymal Stem Cell Growth Medium 2 ([PromoCell (Heidelberg, Germany), C-28009] and MSCGM Mesenchymal Stem Cell Growth Medium [Lonza (Basel, Switzerland), PT-3001] |
| MSC: human bone marrow-derived mesenchymal stem cells [Lonza (Basel, Switzerland), PT-2501] | Mesenchymal Stem Cell Growth Medium [Lonza (Basel, Switzerland), PT-3001] |
| NHDF: normal human adult skin fibroblasts [Lonza (Basel, Switzerland), CC-2511] | FGM™-2 Fibroblast Growth Medium-2 BulletKit™ [Lonza (Basel, Switzerland), CC-3132)] |

Preparation of RetroNectin-coated plate: RetroNectin [Takara Bio Inc. (Shiga, Japan), T100B] was diluted to 40 μg/mL with PBS (Dulbecco's phosphate-buffered saline (Ca, Mg-free)) [Nacalai Tesque (Kyoto, Japan), 14249-95] and added to a 96-well plate with no surface treatment [Corning (Tokyo, Japan), 3370] at 100 μL/well. The plate was then left overnight at 4°C. Before the plate was used, the RetroNectin solution was removed, the plate was washed twice with PBS, and the following operations were performed.

Cell seeding and lentivirus infection: The cells were detached from the plate with Accutase-Solution [PromoCell (Heidelberg, Germany), C-41310] for blister-derived cells, Trypsin/EDTA for Mesenchymal Stem Cells [Lonza (Basel, Switzerland), CC-3232] for human bone marrow-derived mesenchymal stem cells, and Trypsin/EDTA Solution [Lonza (Basel, Switzerland), CC-5012] for normal human adult skin fibroblasts, and collected by using the cell culture medium for respective cells. The number of collected cells was counted and the cells were seeded on the RetroNectin-coated 96-well plate at 2500 cells/well. After that, a lentivirus having a GFP gene: pLenti-C-mGFP [ORIGENE (Rockville, USA), PS100071] was added to each well at MOI 1 or MOI 5, and the cells were cultured in a $CO_2$ incubator for 72 hours.

Detection of GFP-positive cells: The GFP-positive cell ratio was detected with a fluorescence microscope [Keyence (Tokyo, Japan), BZ-X710]. The results are shown in Fig. 9.

Also, GFP-positive cells after infection with MOI 1 were quantified by the following procedure for each cell type. First, the ratio of the number of GFP-positive cells to the total number of cells in a visual field was defined as the GFP-positive cell ratio. This measurement was repeated three times and statistical processing was performed (*: P <0.05, Dunnett's test). The results are shown in Fig. 10.

As shown in Fig. 9, it was found that the blister-derived cells showed a higher GFP-positive cell ratio and a higher GFP fluorescence intensity than the mesenchymal stem cells and fibroblasts. Further, as shown in Fig. 10, the GFP-positive cell ratio of the blister-derived cells was found to be significantly higher than that of the mesenchymal stem cells or fibroblasts. These results suggest that the efficiency of lentiviral gene delivery to the blister-derived cells is higher than those to the mesenchymal stem cells and fibroblasts.

6. Preparation of lentiviral vector plasmid having type VII collagen gene

A lentiviral vector plasmid containing an EF1α promoter and a COL7A1 gene in an expression cassette as shown in Fig. 11 (left) was generated. First, from the Flexi ORF sequence-verified clone (Promega) containing COL7A1 cDNA, the COL7A1 cDNA was cut out by treatment with SpeI and XbaI, which produced a paired sequence, and ligated to pLVSIN-EF1α Puro (Takara Bio) treated with XbaI to produce pLVSIN-EF1α-C7 Puro. The resulting product was further treated with NotI and MluI to remove the PGK-Puro cassette to provide pLVSIN-EF1α-COL7A1.

Also, a lentiviral vector plasmid containing a PGK promoter and a COL7A1 gene in an expression cassette as shown in Fig. 11 (right) was generated. The EF1α promoter region was cut out from pLVSIN-EF1α-Col7A1 by treatment with ClaI and SwaI, and the PGK promoter region (501 bp) was amplified by PCR using AAVS1 hPGK-PuroR-pA donor plasmid (Addgene #22072) as a template, and the PGK promoter region was integrated by Gibson assembly to provide pLVSIN-PGK-COL7A1. The PGK promoter was amplified with KOD One (Toyobo). For the Gibson assembly, NEBuilder HiFi DNA Assembly Kit (New England Biolabs) was used.

7. Production of lentiviral vector having type VII collagen gene

A lentiviral vector having a COL7A1 gene as shown in Fig. 12 was produced.

Transfection using lentiviral plasmids: The plasmids shown in Fig. 11 were used as lentiviral vector plasmids. The packaging plasmid used was Lentiviral High Titer Packaging Mix [Takara Bio (Shiga, Japan), 6194]. First, Lenti-X 293T cells [Takara Bio Inc. (Shiga, Japan), 632180] (cell line for lentivirus packaging) were seeded in a 100 mm dish [Corning (New York, USA), 353003] at 5000000 cells/dish and cultured overnight in a $CO_2$ incubator. The medium for Lenti-X 293T cells was DMEM [Nacalai Tesque (Kyoto, Japan), 08457-55] containing 10% FBS and penicillin/streptomycin (added to final concentrations of 100 unit/mL and 100 μg/mL, respectively). The vector plasmid and the packaging plasmid were then transfected using polyethylenimine. The transfected cells were cultured overnight in a $CO_2$ incubator and then the medium was changed. The transfection reagents used were OPTI-MEM [Thermo Fisher Science (Tokyo, Japan), 31985062] and PEI-MAX [Polysciences (Warrington, USA), 24765-100]. The PEI-MAX recommended protocol was used as a transfection protocol. The recommended protocol of Lentiviral High Titer Packaging Mix was used for the mixing ratio of the vector plasmid and packaging plasmid.

Collection and purification of lentiviral vector: The culture supernatant of Lenti-X 293T cells 72 hours after the transfection was collected and coarsely centrifuged at 300 g for 5 min to remove cell debris. The supernatant was filtered using a 0.45 μm filter [Merck (Tokyo, Japan), SLHVR33RS] to further remove cell debris. The filtered supernatant was then centrifuged (6000g, 4°C, 20hr) to pellet the lentiviral vector and the pellet was resuspended in 1.5 mL of PBS. Next, in an ultracentrifuge tube [Beckman Coulter (Tokyo, Japan), 344058], 55% sucrose/PBS solution (1 mL), 20% sucrose/PBS solution (2.5 mL), and the lentiviral vector solution (1.5 mL) were layered and ultracentrifuged (41000 rpm, 4°C, 2hr). The ultracentrifuge and the rotor used were [Beckman Coulter (Tokyo, Japan), L-90K] and [Beckman Coulter (Tokyo, Japan), SW55Ti]. After ultracentrifugation, the lentiviral vector layer appearing between 55% sucrose/PBS solution and 20% sucrose/PBS solution was collected and diluted to 1 mL with PBS. Next, 20% sucrose/PBS solution (4 mL) and the lentiviral vector solution (1 mL) were layered in an ultracentrifugation tube, and ultracentrifuged again (41000 rpm, 4°C, 2hr). The precipitated lentiviral vector pellet was well suspended in 400 μL of DMEM to obtain a lentiviral vector solution.

The lentiviral vector titer was determined as follows.

Preparation of RetroNectin-coated plate: RetroNectin [Takara Bio Inc. (Shiga, Japan), T100B] was diluted with PBS to 100 μg/mL and added to a flat-bottom 48-well plate with no surface treatment [IWAKI (Tokyo, Japan), 1830-048] at 100 μL/well. The plate was then left overnight at 4°C. Before the plate was used, the RetroNectin solution was removed, and the plate was blocked with 2% FBS-containing PBS at room temperature for 30 minutes, followed by the following procedures.

Cell seeding and lentiviral infection: To the RetroNectin-coated 48-well plate, the LVSIN-EF1α-COL7A1 lentiviral vector or LVSIN-PGK-COL7A1 lentiviral vector (see Fig. 12) was added with a certain volume of virus solution in each well and centrifuged at 2000 g and 32°C for 2 hours. Then, the virus solution was removed and the plate was washed once with PBS. Next, the blister-derived cells were detached from the plate using Accutase-Solution and collected by using a medium. The collected cells were counted and seeded on the RetroNectin-coated 48-well plate at 12500 cells/well. Cells 14 days after the infection were detached from the plate with Accutase-Solution and collected by using a medium. Then, genomic DNA was extracted with Maxwell® RSC Instrument [Promega (Tokyo, Japan), AS4500] and Maxwell® RSC Blood DNA Kit [Promega (Tokyo, Japan), AS 1400]. By using Lenti-X™ Provirus Quantitation Kit [Takara Bio Inc. (Shiga, Japan), 631239], Vector Copy Number (VCN) was calculated from the collected genomic DNA. The virus titer (TU/mL, TU: Transduction Unit) was calculated from the VCN and the volume of virus solution at the time of virus infection. The calculation formula is as follows.

$$Virus\ titer\ (TU/mL)$$

$$= \frac{VCN\ (copies/cell) \times\ Number\ of\ cells\ at\ the\ time\ of\ infection\ (cells)}{Volume\ of\ virus\ solution\ at\ the\ time\ of\ virus\ infection\ (\mu L)}$$

$$\times \frac{1000\ \mu L}{1\ mL}$$

Based on the virus titer thus obtained, the volume of virus solution required for setting the Multiplicity Of Infection (MOI) in the lentivirus infection experiment was calculated.

8. Analysis of type VII collagen gene transfer efficiency by immunostaining

We analyzed the efficiency of type VII collagen gene transfer by lentiviral vectors to blister-derived cells.

Preparation of RetroNectin-coated plate: A plate was coated with RetroNectin in the same manner as in "7. Production of lentiviral vector having type VII collagen gene".

Cell seeding and lentiviral infection: To the RetroNectin-coated 48-well plate, the LVSIN-EF1$\alpha$-COL7A1 lentiviral vector or LVSIN-PGK-COL7A1 lentiviral vector (see Fig. 12) was added at MOI 0.5, MOI 1, or MOI 2 in each well and centrifuged at 2000 g and 32°C for 2 hours. Then, the virus solution was removed and the plate was washed once with PBS. Next, the blister-derived cells were detached from the plate using Accutase-Solution and collected by using a medium. The collected cells were counted and seeded on the RetroNectin-coated 48-well plate at 12500 cells/well.

Immunostaining: The blister-derived cells 14 days after the lentivirus infection were detached from the plate with Accutase-Solution and collected by using a medium. The collected cells were counted, seeded on a CC2-coated chamber slide [Thermo Fisher Science (Tokyo, Japan), 154852] at 50000 cells/well, and cultured in a $CO_2$ incubator. After 24 hours, immunostaining was performed by using an anti-type VII collagen antibody (clone LH7.2) [Sigma Aldrich (Tokyo, Japan), C6805] and Alexa488 [Thermo Fisher Science (Tokyo, Japan), A-11001]. Then, the expression of type VII collagen was analyzed with a confocal fluorescence microscope [Nikon (Tokyo, Japan), Nikon A1R HD25].

Results are shown in Figs. 13 and 14. Infection of cells with the lentiviral vector having either EF1$\alpha$ promoter or PGK promoter increased the expression of type VII collagen in an MOI-dependent manner. The highest ratio of type VII collagen-positive cells was observed in the cells infected at MOI 2 with any of the vectors, approximately 30% with the LVSIN-EF1$\alpha$-COL7A1 lentiviral vector and approximately 16% with the LVSIN-PGK-COL7A1 lentiviral vector. Also, in general, blister-derived cells infected with the LVSIN-EF1$\alpha$-COL7A1 lentiviral vector showed a higher ratio of type VII collagen-positive cells and stronger staining intensity than the blister-derived cells infected with the LUSIN-PGK-COL7A1 lentiviral vector.

9. Analysis of type VII collagen gene transfer efficiency by flow cytometry (FACS)

We analyzed the efficiency of type VII collagen gene transfer by lentiviral vectors to the blister-derived cells.

Preparation of lentivirus-infected cells: The cells were prepared in the same manner as described in "8. Analysis of type VII collagen gene transfer efficiency by immunostaining".

FACS analysis: Blister-derived cells 14 days after the lentivirus infection were detached from the plate with Accutase-Solution and collected by using a medium. The number of collected cells were measured and the cells were divided to 300000 cells/sample and permeabilized by using eBioscience™ Permeabilization Buffer [Thermo Fisher Science (Tokyo, Japan), 00-8333-56]. Then, immunostaining was performed by using an anti-type VII collagen antibody (clone LH7.2) [Sigma Aldrich (Tokyo, Japan), C6805] and Alexa488 [Thermo Fisher Science (Tokyo, Japan), A-11001), and the expression of type VII collagen was analyzed by a flow cytometer (BD FACSCanto™ II) [Nippon Becton Dickinson (Tokyo, Japan)].

The results are shown in Fig. 15. The percentage of the cells contained in the encircled region in each FACS data is shown in Fig. 16 (left) as the percentage of type VII collagen-positive cells. Further, the Mean Fluorescence Intensity (MFI) within the encircled region is shown in Fig. 16 (right) as the mean fluorescence intensity of type VII collagen-positive cells. As shown in Fig. 15 and Fig. 16 (left), the percentage of type VII collagen-positive cells increased in an MOI-dependent manner. The highest percentage of type VII collagen-positive cells was observed with MOI 2 infection, approximately 18% with EF1$\alpha$-COL7A1 lentivirus and approximately 12% with PGK-COL7A1 lentivirus. Also, as shown in Fig. 16 (right), the MFI of EF1$\alpha$-COL7A1 lentivirus-infected cells was about 2.2 times higher than that of PGK-COL7A1 lentivirus-infected cells. These results suggest that the EF1$\alpha$ promoter induces expression of a higher amount of COL7A1 than the PGK promoter in the blister-derived cells transduced with a lentiviral vector.

Also, as a preliminary study, blister-derived cells, human bone marrow-derived mesenchymal stem cells, and normal human adult skin fibroblasts were infected with the EF1$\alpha$-COL7A1 lentiviral vector or PGK-COL7A1 lentiviral vector, and immunostaining and flow cytometry (FACS) were performed in the same manner as above to measure the percentage of type VII collagen-positive cells. The blister-derived cells showed a higher percentage of type VII collagen-positive cells than the human bone marrow-derived mesenchymal stem cells and normal human adult skin fibroblasts.

10. Vector Copy Number (VCN) of lentivirus-infected blister-derived cells

VCN of the blister-derived cells infected with the lentiviral vector having the type VII collagen gene was analyzed. First, the blister-derived cells were infected with a lentiviral vector in the same manner as in "8. Analysis of type VII collagen gene transfer efficiency by immunostaining". The cells 7, 14, 21, and 28 days after the infection were detached from the plate with Accutase-Solution and collected by using a medium. Next, from the collected cells, genomic DNA was extracted by using Maxwell® RSC Instrument [Promega (Tokyo, Japan), AS4500] and Maxwell® RSC Blood DNA Kit [Promega (Tokyo, Japan), AS 1400]. VCN was calculated from the collected genomic DNA by using the Lenti-X™ Provirus Quantitation Kit [Takara Bio Inc. (Shiga, Japan), 631239].

The results are shown in Fig 17. As shown in the figure, VCN increased in an MOI-dependent manner. Also, VCN decreased as the days passed after the infection, but stabilized after the day 21. These results suggest that the type VII collagen gene inserted into the genome of blister-derived cells by a lentiviral vector can be maintained in the genome for a long period of time.

11. Production of cell sheets

Blister fluid from a patient with dystrophic epidermolysis bullosa was collected. The obtained solution was inoculated in a collagen I-coated 6-well plate. The medium used was an equal-volume mixed medium of Mesenchymal Stem Cell Growth Medium 2 (PromoCell, C-28009) and MSCGM Mesenchymal Stem Cell Growth Medium (Lonza, PT-3001) to which penicillin and streptomycin were added to be final concentrations of 50 units/mL and 50 $\mu$g/mL, respectively. The plate was incubated at 37°C, 5% $CO_2$ to obtain adherent cells at the bottom of the plate. The blister-derived cells thus obtained were then expanded to the desired cell number though medium exchange and passaging as appropriate.

Then, in the same manner as in "4. Introduction of COL7A1 gene into blister-derived cells", a COL7A1 gene was introduced into the blister-derived cells. The promoter used was a CAG promoter. The gene-introduced blister-derived cells were then expanded to the desired cell number though medium exchange and passaging as appropriate.

The gene-introduced blister-derived cells were seeded in a 12-well plate at $0.4\times10^6$ cells/cm$^2$ (corresponding to a confluency of 700%). The medium used was an equal-volume mixed medium of Mesenchymal Stem Cell Growth Medium 2 (PromoCell, C-28009) and MSCGM Mesenchymal Stem Cell Growth Medium (Lonza, PT-3001). The plate was incubated at 37°C, 5% $CO_2$ for 2 days. As a resultl, a cell sheet was formed at the bottom of the 12-well plate. A $1 \times 1$ cm square slit was made in the cell sheet with a blade, and the cell sheet was detached from the bottom of the plate with tweezers. The cell sheet in the middle of being detached from the plate is shown in Fig. 18 (top left). The cell sheet removed from the plate is shown in Fig. 18 (top middle).

Fig. 18 (top right) shows a photograph of hematoxylin-eosin staining (HE staining) of the obtained cell sheet. As shown in the figure, in the obtained cell sheet, the cells overlapped each other in the thickness direction of the sheet. In other words, the cells formed multiple layers in the obtained cell sheet. A detailed observation of the HE staining revealed that the cell density near the surface of the sheet was high, while in the interior, especially in the central portion of the sheet, the cell density was not as high as near the surface. The thickness of the obtained cell sheet was 15 to 20 $\mu$m.

As a preliminary study, the gene-introduced blister-derived cells were seeded in a 12-well plate at $0.1\times10^6$ cells/cm$^2$ (corresponding to a confluency of 175%) or $0.2\times10^6$ cells/cm$^2$ (corresponding to a confluency of 350%). When the cells were seeded at $0.2\times10^6$ cells/cm$^2$, a cell sheet was formed, but the cell sheet had inferior mechanical strength and slightly more easily broke than the cell sheet seeded at $0.4\times10^6$ cells/cm$^2$. A cell sheet was also formed when the cells were seeded at $0.1\times10^6$ cells/cm$^2$, but the cell sheet had more inferior mechanical strength and easily broke, and it was thus difficult to handle it compared to the cell sheet seeded at $0.2\times10^6$ cells/cm$^2$. Also, the gene-introduced blister-derived cells were seeded in a 12-well plate at $0.4\times10^6$ cells/cm$^2$ and cultured for one to three days. When the cells were cultured for one day, a cell sheet was formed, but the cell sheet had inferior mechanical strength and more easily broke than the cell sheet cultured for two days. The strength of the cell sheet was slightly higher when cultured for three days than when cultured for two days.

12. Application of the cell sheet to a skin lesion

As shown in Fig. 18 (bottom left), the cell sheet obtained in "11. Production of cell sheets" was transplanted onto the skin lesion of an epidermolysis bullosa model mouse. First, the whole skin layer of a Col7Aa1 gene knockout neonatal mouse (Col7a1-/-), which showed blister formation, was excised out, and transplanted onto the back of an immunodeficient mouse (NOD-SCID). Nine to ten days after the transplantation, an epidermolysis bullosa model mouse was obtained, as shown in Fig. 18 (second from the left at the bottom), in which the transplanted skin fragment derived from the knockout mouse was engrafted. Next, as shown in Fig. 18 (second from the right at the bottom), a scalpel was used to make cuts on three sides of a 1 × 1 cm square in the epidermis of the lesion to peel off the epidermis and expose the dermis. The cell sheet was then applied to the exposed dermis, as shown in Fig. 18 (bottom right). Finally, the cut epidermis was put back in place and fixed with a medical tape. As a comparative control, a suspension of blister-derived cells, into which a COL7A1 gene was introduced by the same procedure as in "4. Introduction of COL7A1 gene into blister-derived cells", was administered intradermally into the dermis.

Four weeks after the transplantation of the cell sheet, the skin in the transplanted area was collected and immunostained with an anti-type VII collagen antibody (clone LH7.2; Sigma Aldrich, C6805), and the deposit of type VII collagen on the basement membrane was analyzed using a confocal fluorescence microscope [Nikon, Tokyo, Japan, Nikon A 1R HD25]. The results are shown in Figs. 19 to 21. In addition, 7 days after the transplantation of the cell sheet, multiple staining with in situ hybridization (ISH) using a probe that binds specifically to human COL7A1 mRNA but not to mouse Col7a1 mRNA and immunostaining for human type VII collagen protein was performed according to TECHNICAL NOTE of RNAscope® RED Assay and Immunofluorescence (Advanced Cell Diagnostics). The ISH probe targeted NM_000094.4, 1510-4172, and was designed and manufactured by Advanced Cell Diagnostics (Newark, CA). The ISH of the multiple staining was performed using RNAscope® 2.5 HD Detection Reagents-RED (Advanced Cell Diagnostics, 322360). The immunostaining was performed using an anti-type VII collagen antibody (Atlas Antibodies, HPA042420). The results are shown in Figs. 22 and 23. Also, 32 days after the transplantation of the cell sheet, the skin of the transplanted area was observed under an electron microscope. The preparation of the samples for electron microscopic observation including chemical fixation and resin-embedded ultrathin sectioning, and as well as the observation and imaging by transmission electron microscopy were performed by Tokai Electron Microscopy, Inc. (Nagoya, Japan). The results are shown in Fig. 24.

As shown in Figs. 19 and 20, type VII collagen deposit was observed around the basement membrane over a very long distance in the skin onto which the cell sheet was transplanted. In contrast, in the skin that received the intradermal administration, type VII collagen deposit was observed in the deep dermis, and little deposit of type VII collagen was observed around the basement membrane. The fluorescence intensity of immunostaining for type VII collagen at the basement membrane of the skin onto which the cell sheet was transplanted was quantified and found to be higher than that in the healthy human skin, as shown in Fig. 21.

As shown in Fig. 22, human COL7A1 mRNA was detected just beneath the epidermis of the skin onto which the cell sheet was transplanted. This suggests that the cells derived from the cell sheet remained near the dermal surface. As shown in Fig. 23, the cells derived from the cell sheet formed a thick layer near the dermal surface from place to place. In the epidermis, old cells are lost and gradually replaced by new cells. On the other hand, inside the dermis, cells are known to remain for a long period of time and maintain skin functions. The fact that the transplanted cells remain in the dermis with thickness suggests that these cells can remain in the dermis for a long period of time and provide long-lasting medicinal effects.

As shown in Fig. 24, anchoring fibrils were formed around the basement membrane of the skin onto which the cell sheet was transplanted. In contrast, anchoring fibrils did not form in the skin onto which the cell sheet was not transplanted.

13. Production of cell sheets comprising type VII collagen gene introduced by lentiviral vector

The lentiviral vector produced in "7. Production of lentiviral vector having type VII collagen gene" was used to introduce the type VII collagen gene into the blister-derived cells obtained in "11. Production of cell sheets". Specifically, blister-derived cells were infected with the LVSIN-EF1α-COL7A1 or LVSIN-PGK-COL7A1 lentiviral vector at MOI 2 according to the same procedure as described in "8. Analysis of type VII collagen gene transfer efficiency by immunostaining". The obtained lentivirus-infected blister-derived cells were used to produce cell sheets as described in "11. Production of cell sheets". Then, the cell sheet thus produced was transplanted onto the lesion of an epidermolysis bullosa model mouse in the same manner as in "12. Application of the cell sheet to a skin lesion". As a comparative control, $0.4 \times 10^6$ cells of the LVSIN-EF1α-COL7A1 lentivirus-infected blister-derived cells obtained above were seeded at normal passage density (corresponding to confluency of 10% to 20%). After 2 days of culture at 37°C, 5% $CO_2$, the cells were detached using Accutase-Solution and the obtained cell suspension was administered intradermally into the dermis of a skin lesion.

Four weeks after the transplantation, the skin in the transplanted area was collected and immunostained with an anti-type VII collagen antibody (clone LH7.2; Sigma Aldrich, C6805) to examine the deposit of type VII collagen at the basement membrane. The results are shown in Figs. 25 and 26. As shown in Fig. 25 and Fig. 26 left, deposit of type VII collagen was negligible at the basement membrane of the skin into which the suspension of the blister-derived cells was intradermally administered. In contrast, with the blister-derived cell sheet transduced with the LVSIN-PGK-COL7A1 lentiviral vector, deposit of type VII collagen was observed along the basement membrane. Also, as shown in Fig. 25 and Fig. 26 right, the fluorescence intensity of immunostaining for type VII collagen was higher with the blister-derived cell sheet transduced with the LVSIN-PGK-COL7A1 lentiviral vector than with the cell suspension. As shown in Fig. 25 and Fig. 26 left, with the blister-derived cell sheet transduced with the LVSIN-EF1$\alpha$-COL7A1 lentiviral vector, deposit of type VII collagen was observed over a very long distance along the basement membrane. Also, as shown in Fig. 25 and Fig. 26 right, the fluorescence intensity of immunostaining for type VII collagen was high with the blister-derived cell sheet transduced with the LVSIN- EF1$\alpha$-COL7A1 lentiviral vector. Based on these results, blister-derived cell sheets transduced with the LVSIN-PGK-COL7A1 lentiviral vector are expected to have higher therapeutic efficacy than cell suspensions. In addition, blister-derived cell sheets transduced with the LVSIN-EF1$\alpha$-COL7A1 lentiviral vector are expected to have even higher therapeutic efficacy.

**Claims**

1. A cell sheet for transplantation onto a skin of a patient with dystrophic epidermolysis bullosa, comprising cells which are derived from a body fluid within a blister of a patient with dystrophic epidermolysis bullosa and into which a type VII collagen gene is introduced.

2. The cell sheet according to claim 1, wherein

   a type VII collagen gene expression cassette is introduced into the cells, and
   the type VII collagen gene expression cassette comprises an EF1$\alpha$ promoter and the type VII collagen gene located downstream of the EF1$\alpha$ promoter.

3. The cell sheet according to claim 1, wherein the cells overlap each other in a thickness direction of the sheet.

4. The cell sheet according to claim 3, wherein, in a longitudinal section of the cell sheet, the cell sheet has a higher cell density near a surface of the sheet than in a central portion of the sheet.

5. The cell sheet according to claim 1, wherein the cell sheet is transplanted onto a part of the skin that lacks epidermis.

6. A method for producing a cell sheet for transplantation onto a skin of a patient with dystrophic epidermolysis bullosa, comprising the steps of:

   inoculating at least a portion of a body fluid within a blister of a patient with dystrophic epidermolysis bullosa into a medium,
   obtaining cells adhering to a bottom of a culture vessel,
   introducing a type VII collagen gene into the cells, and
   culturing the cells comprising the introduced type VII collagen gene, and
   taking out the cultured cells from a culture vessel as the cell sheet.

7. The method for producing a cell sheet according to claim 6, comprising inoculating cells obtained from the body fluid within the blister into the medium without enzymatic treatment.

8. The method for producing a cell sheet according to claim 6, wherein the type VII collagen gene is introduced into the cells with a lentiviral vector.

9. The method for producing a cell sheet according to claim 6, further comprising the step of seeding the cells comprising the introduced type VII collagen gene to be overconfluent.

10. The method for producing a cell sheet according to claim 6, further comprising the step of seeding the cells comprising the introduced type VII collagen gene into a culture vessel at a cell density of $1\times10^4$ cells/cm$^2$ or higher.

11. The method for producing a cell sheet according to claim 6, comprising taking out the cultured cells from the culture vessel as the cell sheet without enzymatic treatment.

[Fig. 1]

```
1              Collection of a blister fluid
                        ↓
2      Inoculation of the blister fluid and culturing
                        ↓
3            Passage of the adherent cells
                        ↓
4                   Gene transfer
                        ↓
5        Passage of the gene-transferred cells
                        ↓
6        Seeding of the gene-transferred cells
                        ↓
7            Collection of the cell sheet
```

[Fig. 2]

[Fig. 3]

[Fig. 4]

[Fig. 5]

**Lysate**

**Medium**

[Fig. 6]

[Fig. 7]

[Fig. 8]

[Fig. 9]

[Fig. 10]

[Fig. 11]

[Fig. 12]

**LVSIN-EF1α-COL7A1**

| 5' LTR | ψ | RRE | cPPT /CTS | pEF1α | COL7A1 | WPRE | 3' LTR /ΔU3 |
|---|---|---|---|---|---|---|---|

**LVSIN-PGK-COL7A1**

| 5' LTR | ψ | RRE | cPPT /CTS | pPGK | COL7A1 | WPRE | 3' LTR /ΔU3 |
|---|---|---|---|---|---|---|---|

40

[Fig. 13]

[Fig. 14]

[Fig. 15]

[Fig. 16]

Percentage of C7-positive cells

Mean fluorescence intensity
of C7-positive cells

[Fig. 17]

EP 4 467 646 A1

EF1a-C7

PGK-C7

[Fig. 18]

[Fig. 19]

[Fig. 20]

[Fig. 21]

Fluorescence intensity

[Fig. 22]

[Fig. 23]

Bar = 500 μm

[Fig. 24]

No cell sheet transplantation / Cell sheet transplantation / Cell sheet transplantation / Low magnification / High magnification

[Fig. 25]

LVSIN-EF1α-COL7
cell sheet

LVSIN-PGK-COL7
cell sheet

LVSIN-EF1α-COL7
cell suspension

[Fig. 26]

Length of COL7 deposit
at the basement membrane
(μm)

Mean fluorescence intensity
in the COL7 deposit area

# INTERNATIONAL SEARCH REPORT

| | International application No. |
|---|---|
| | **PCT/JP2023/001669** |

## A. CLASSIFICATION OF SUBJECT MATTER

*C12N 15/12*(2006.01)i; *A61K 35/36*(2015.01)i; *A61L 27/38*(2006.01)i; *A61L 27/50*(2006.01)i; *A61L 27/60*(2006.01)i; *A61P 17/04*(2006.01)i; *C12N 5/071*(2010.01)i; *C12N 5/10*(2006.01)i; *C12N 15/63*(2006.01)i; *C12N 15/867*(2006.01)i
FI: C12N15/12; A61K35/36; A61L27/38 300; A61L27/50; A61L27/60; A61P17/04; C12N5/071 ZNA; C12N5/10; C12N15/867 Z; C12N15/63 Z

According to International Patent Classification (IPC) or to both national classification and IPC

## B. FIELDS SEARCHED

Minimum documentation searched (classification system followed by classification symbols)

C12N15/12; A61K35/36; A61L27/38; A61L27/50; A61L27/60; A61P17/04; C12N5/071; C12N5/10; C12N15/63; C12N15/867

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

## C. DOCUMENTS CONSIDERED TO BE RELEVANT

| Category* | Citation of document, with indication, where appropriate, of the relevant passages | Relevant to claim No. |
|---|---|---|
| X | JP 2019-506925 A (THE BOARD OF TRUSTREES OF THE LELAND STANFORD JUNIOR UNIVERSITY) 14 March 2019 (2019-03-14) claims 1, 3, 9, paragraphs [0056], [0119]-[0127] | 1, 3-5 |
| Y | claims 1, 3, 9, paragraphs [0056], [0119]-[0127] | 2 |
| A | claims 1, 3, 9, paragraphs [0056], [0119]-[0127] | 6-11 |
| X | WO 2018/154413 A1 (CRISPR THERAPEUTICS AG) 30 August 2018 (2018-08-30) claims 3-7, 15-17, 19, 22-25, 27, 33 | 1, 3-5 |
| A | claims 3-7, 15-17, 19, 22-25, 27, 33 | 2, 6-11 |
| Y | WO 2020/149395 A1 (OSAKA UNIVERSITY) 23 July 2020 (2020-07-23) claim 1, paragraph [0038] | 2 |
| A | claim 1, paragraph [0038] | 1, 3-11 |
| P, Y | WO 2022/019325 A1 (OSAKA UNIVERSITY) 27 January 2022 (2022-01-27) claims 1-15 | 1-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 March 2023** | **20 March 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2023/001669**

**C.    DOCUMENTS CONSIDERED TO BE RELEVANT**

| Category* | Citation of document, with indication, where appropriate, of the relevant passages | Relevant to claim No. |
|---|---|---|
| P, Y | WO 2022/018884 A1 (OSAKA UNIVERSITY) 27 January 2022 (2022-01-27)<br>claims 1-14 | 1-11 |

Form PCT/ISA/210 (second sheet) (January 2015)

**INTERNATIONAL SEARCH REPORT**

International application No.

**PCT/JP2023/001669**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    "The form of Annex C/ST.25 text file" above shall read as "the form of ST.26.".

Form PCT/ISA/210 (continuation of first sheet) (January 2015)

**INTERNATIONAL SEARCH REPORT**
Information on patent family members

International application No.

**PCT/JP2023/001669**

| Patent document cited in search report | | | Publication date (day/month/year) | Patent family member(s) | | | Publication date (day/month/year) |
|---|---|---|---|---|---|---|---|
| JP | 2019-506925 | A | 14 March 2019 | US 2019/0382724 A1<br>claims 1, 3, 9, paragraphs [0072], [0131]-[0139]<br>WO 2017/120147 A1<br>EP 3400287 A1<br>CN 108473952 A | | | |
| WO | 2018/154413 | A1 | 30 August 2018 | US 2019/0365929 A1<br>claims 3-7, 15-17, 19, 22-25, 27, 33<br>EP 3585897 A1 | | | |
| WO | 2020/149395 | A1 | 23 July 2020 | US 2022/0088083 A1<br>claim 1, paragraph [0048]<br>EP 3912644 A1<br>KR 10-2021-0116531 A<br>CN 113646006 A | | | |
| WO | 2022/019325 | A1 | 27 January 2022 | (Family: none) | | | |
| WO | 2022/018884 | A1 | 27 January 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022008057 A
- WO 2017120147 A
- WO 2018154413 A
- JP 7014249 A
- US 100071 A